# EUROPEAN PATENT SPECIFICATION

(11) **EP 0 983 074 B1**
(45) Date of publication and mention of the grant of the patent: **30.07.2003**
(21) Application number: 98926059.1
(22) Date of filing: 22.05.1998
(51) Int. Cl.: A61K 35/14

(54) **NON-MYELOABLATIVE TOLEROGENIC TREATMENT**
NON MYELOABLATIVE THERAPIE FÜR TOLERANZINDUKTION
TRAITEMENT TOLEROGENIQUE NON MYELOABLATIF

(30) Priority: 23.05.1997 US 862550
(43) Date of publication of application: 08.03.2000
(73) Proprietor: HADASIT MEDICAL RESEARCH SERVICES AND DEVELOPMENT LTD., 91120 Jerusalem (IL); BAXTER INTERNATIONAL INC., Deerfield, Illinois 60015 (US)
(72) Inventor: SLAVIN, Shimon, Jerusalem (IL); PRIGOZHINA, Tatyana, Rehovot (IL)
(74) Representative: Bassett, Richard Simon
(86) International application number: US9810575
(87) International publication number: WO98052582

(56) References cited:
- WO-A-93/13785
- WO-A-95/03062
- WO-A-96/37208
- US-A- 5 273 738
- US-A- 5 514 364
- US-A- 5 635 156
- Nature Medicine, Volume 1, No. 12, December 1995, Esther Bachar-Lustig et al, "Megadose of T cell-depleted bone marrow overcomes MHC barriers in sublethally irradiated mice"
- Immunology Today, Volume 16, No. 9, 1995, Yair Reisner et al, "Bone marrow transplantation across HLA barriers by increasing the number of transplanted cells"
- Ann Thorac Surg, Volume 56, 1993, David H. Sachs, "Transplantation Tolerance"
- Immunological Reviews, Volume 157, 1997, Robertson Parkman et al, "Immunological reconstitution following bone marrow transplantation"
- Chemical Abstracts, volume 124, no 7, 12 February 1996, (Columbus, Ohio, US), Bachar-Lustig, Esther et al, "Megadose of T cell-depleted bone marrow overcomes MHC barriers in sublethally irradiated mice",, THE ABSTRACT No 80953, Nat.Med.(N.Y.) 1995, 1 12), 1268-1273
- Chemical Abstracts, volume 126, no 24, 10 June 1996, (Columbus, Ohio, US), Scheding, Stefan et al, "Transplantation of ex-vivo expanded peripheral blood progenitor cells after high dose chemotherapy in cancer patients",, THE ABSTRACT No 325042, Bone Marrow Transplant:Basic Clin.Stud. 1996, 250-257
- Transplantation, Volume 60, No. 7, October 1995, Elizabeth H. Field et al, "Alloantigen Priming After Total Lymphoid Irradiation Alters Alloimmune Cytokine Responses"
- Diabetes, Volume 45, February 1996, John A. Goss et al, "Induction of Allogeneic Islet Survival by Intrahepatic Islet Preimmunization and Transient Immunosuppression"
- Chemical Abstracts, volume 126, no 11, 17 March 1997, (Columbus, Ohio, US), Ran, Xinze et al, "Effects of blood transfusion on bone marrow transplantation for the survival of rats after total lymphoid irradiation treatied with cyclophosphamide",, THE ABSTRACT No 141429, J.Med.Coll. 1996, 11 3), 227-230
- Blood, Volume 77, No. 11, June 1991, Robert Truitt et al, "Impact of Pretransplant Conditioning and Donor T Cells on Chimerism, Graft-Versus-Host Disease, Graft-Versus-Leukemia Reactivity, and Tolerance After Bone Marrow Transplantation"

## Description

### Background of the Invention

Transplantation of organs, hematopoietic cells and somatic cells has been a crucial therapeutic regimen for patients suffering from a variety of maladies. Although the techniques necessary for transplants are quite straight-forward, the great stumbling block for successful transplantation has been the immune system. A fundamental problem has been the great vigor with which the host immune system reacts against introduction of antigens found in donor tissues or cells.

Transplantation of allogeneic donor (i.e., the same species but not genetically identical to the host patient) or xenogeneic donor (i.e., a species other than that of the host) grafts has posed particularly great difficulties. The continued functioning of any donor graft depends upon continued functioning of the donor cells that make up that graft. The cells of donor grafts, however, can elicit an immune reaction on the part of the host which, if unchecked, may lead to destruction of the graft.

One method of alleviating the reaction by the host against a graft has been administration of immunosuppressive treatment to the host. Unfortunately, despite the availability of new and very effective immunosuppressive drugs, recurrent episodes of acute and chronic graft rejection remain common, frequently causing loss of graft function. Moreover, the long-term success of transplantation is often limited by complications resulting from drug-related toxicity and from long-term immunosuppression (e.g. infections and secondary malignancies). In addition, transplantation of bone marrow cells (BMC) or small intestine, which are rich in immunocompetent lymphocytes, frequently is associated with a potential life-threatening complication due to graft versus host disease (GVHD).

It has been shown that a full hematopoietic chimera, i.e., a patient whose own BMC have been 100% replaced by permanently engrafted BMC from another individual (donor), can permanently accept donor-derived allografts with no need for maintenance immunosuppressive therapy. However, induction of full hematopoietic chimerism has been difficult to accomplish. First, substantially complete destruction of the host's immunohematopoietic compartment ("lethal" conditioning) is usually required for engraftment of matched and especially mismatched BMC. With lethal conditioning of the host, GVHD consistently causes morbidity or mortality. In such cases, T cell depletion of the graft hematopoietic material represents the only approach for effective prevention of GVHD. T cell depletion in turn is associated with an increased incidence of graft rejection. To overcome the problem of graft rejection, recipients of T cell depleted marrow allografts may require particularly strong conditioning or, alternatively, very high numbers of T cell depleted BMC. Subjecting patients to aggressive rejection-prevention protocols, such as total body irradiation (TBI) alone or TBI in combination with a short course of immunosuppressive drugs is unlikely to be accepted by clinicians treating patients in need of organ allografts.

It has been proposed that true bilateral tolerance associated with mixed donor/recipient hematopoietic chimerism, i.e., the condition in which a patient possesses both recipient (host) and donor hematopoietic stem cells, rather than with full chimerism, would be preferable in clinical organ transplantation. Several experimental protocols have been designed to induce transplantation tolerance leading to mixed chimerism. Conditioning has required the use of high dose TBI followed by infusion with a mixture of T cell depleted donor and recipient BMC (Sachs et al., Ann. Thorac. Surg., 56:1221 (1993); Ildstad et al., Nature, 307:168 (1984)) or inoculation with donor BMC after lower dose TBI and infusion of a mixture of antibodies against CD4⁺ T cells, CD8⁺ T cells and NK cells leading to general pancytopenia. Tomita et al., J. Immunol., 153:1087 (1994); Tomita et al., Transplantation, 61:469 (1996). An alternative approach has been developed recently involving irradiation with a sublethal dose of TBI and inoculation with a very high number of T cell depleted donor-derived hematopoietic cells. Reisner et al., Immunol. Today, 16:437 (1995); Bachar-Lustig et al., Nature Medicine, 12:1268 (1986). Tolerogenic treatments using cyclophosphamide (hereinafter also referred to as "Cytoxan" or "Cy") in combination with TBI have also been described.

Total lymphoid irradiation (TLI) has been employed successfully as the sole preparatory regimen prior to infusion with donor BMC, to induce mixed hematopoietic chimerism and bilateral transplantation tolerance. Slavin S., Immunol. Today, 3:88 (1987); Slavin et al., Isr. J. Med. Sci., 22:264 (1986). TLI is non-myeloablative and routinely given safely on an outpatient basis to transplant recipients and patients with Hodgkin's disease. Unfortunately, consistent induction of chimerism using TLI has required very high cumulative doses of radiation (3,400-4,400 cGy) which again would not be desirable for transplant recipients. TLI has significant advantages over TBI, especially in the clinical setting. TLI, which involves selective irradiation of the lymphoid compartment without exposing the whole body to ionizing irradiation, is well tolerated. In addition, TLI preserves intact a significant portion of the host's immunohematopoietic system, with resultant retained memory to recall antigens including infective agents. However, long courses of TLI can be time consuming and may be associated with short and long-term side effects that may not be suitable for routine clinical application.

### Summary of the Invention

In one aspect of the present invention there is provided the use of donor antigens from a non-syngeneic donor in the manufacture of a medicament for inducing stable and donor-specific tolerance to non-syngeneic transplants in a host by:
(a) administering said medicament to a host mammal;
(b) administering a non-myeloablative dose of a lymphocytotoxic or tolerizing agent to said host mammal to selectively eliminate said host mammal's lymphocytes responding to said donor antigens; and
(c) administering a preparation of hematopoietic stem cells from said non-syngeneic donor to said host mammal, wherein when said hematopoietic stem cells are stem cells obtained from the liver or yolk sac of embryos, then said embryos are non-human animal embryos.
The invention also provides:
- the use of a lymphocytotoxic or tolerizing agent in the manufacture of a medicament for inducing stable and donor-specific tolerance to non-syngeneic transplants in a host by:
   (a) administering donor antigens from a non-syngeneic donor to a host mammal;
   (b) administering a non-myeloablative dose of said medicament to said host mammal to selectively eliminate said host mammal's lymphocytes responding to said donor antigens; and
   (c) administering a preparation of hematopoietic stem cells from said non-syngeneic donor to said host mammal, wherein when said hematopoietic stem cells are stem cells obtained from the liver or yolk sac of embryos, then said embryos are non-human animal embryos.
- the use of hematopoietic stem cells from a non-syngeneic donor in the manufacture of a medicament for inducing stable and donor-specific tolerance to non-syngeneic transplants in a host by:
   (a) administering donor antigens from said non-syngeneic donor to said host mammal;
   (b) administering a non-myeloablative dose of a lymphocytotoxic or tolerizing agent to said host mammal to selectively eliminate said host mammal's lymphocytes responding to said donor antigens; and
   (c) administering said medicament to said host mammal, wherein when said hematopoietic stem cells are stem cells obtained from the liver or yolk sac of embryos, then said embryos are non-human animal embryos.
- a host derived hematopoietic cell composition comprising host-originating and donor-originating hematopoietic cells, said composition being depleted of donor-specific, host-originating lymphocytes, and said composition being obtainable by a method comprising:
   (a) administering donor antigens from a non-syngeneic donor to a host mammal;
   (b) administering a non-myeloablative dose of lymphocytotoxic or tolerizing agent to said host mammal to selectively eliminate said host mammal's lymphocytes responding to said donor antigens;
   (c) administering a preparation of hematopoietic stem cells from said non-syngeneic donor to said host mammal; and
   (d) isolating said hematopoietic cell composition from said host mammal, wherein when said hematopoietic stem cells are stem cells obtained from the liver of yolk sac of embryos, then said embryos are non-human animal embryos.
- a non-human mammal/human chimera obtainable by a method comprising:
   (a) administering antigens from a human donor to a host non-human mammal;
   (b) administering a non-myeloablative dose of lymphocytotoxic or tolerizing agent to said host mammal to selectively eliminate said host mammal's lymphocytes responding to said antigens; and
   (c) administering a preparation of hematopoietic stem cells from said human donor to said host mammal, wherein said hematopoietic stem cells from said human donor are not stem cells obtained from the liver or yolk sac of embryos.
- a rodent stably engrafted with human hematopoietic stem cells, said rodent constituting a hematopoietic mixed chimera, wherein said hematopoietic mixed chimera is obtainable by a method comprising:
   (a) administering antigens from a human donor to an individual rodent;
   (b) administering a non-myeloablative dose of a lymphocytotoxic or tolerizing agent to said individual rodent to selectively eliminate said individual rodent's lymphocytes responding to said antigens; and
   (c) administering a preparation of hematopoietic stem cells from said human donor to said individual rodent to create said mixed chimera,
   wherein said mixed chimera comprises hematopoietic cells originating from said individual rodent and from said human donor and said hematopoietic cells originating from said individual rodent are depleted of lymphocytes specific for antigens of said human donor, and wherein said hematopoietic stem cells from said human donor are not fetal stem cells obtained from the liver or yolk sac of embryos.
- a pig stably engrafted with human hematopoietic stem cells, said pig constituting a hematopoietic mixed chimera, wherein said hematopoietic mixed chimera is obtainable by a method comprising:
   (a) administering antigens from a human donor to an individual pig;
   (b) administering a non-myeloablative dose of a lymphocytotoxic or tolerizing agent to said individual pig to selectively eliminate said individual pig's lymphocytes responding to said antigens; and
   (c) administering a preparation of hematopoietic stem cells from said human donor to said individual pig to create said mixed chimera,
wherein said mixed chimera comprises hematopoietic cells originating from said individual pig and from said human donor and said hematopoietic cells originating from said individual pig are depleted of lymphocytes specific for antigens of said human donor, and wherein said hematopoietic stem cells from said human donor are not stem cells obtained from the liver or yolk sac of embryos; and
the use of an immunosuppresive agent in the preparation of a medicament, for inducing stable and donor-specific tolerance to non-syngeneic transplants in a host by:
(a) administering said medicament to said host mammal in a non-myeloablative regimen sufficient to decrease said host mammal's functional T lymphocyte population;
(b) administering donor antigens from a non-syngeneic donor to said host mammal;
(c) administering a non-myeloablative dose of a lymphocytotoxic or tolerizing agent to said host mammal to selectively eliminate said host mammal's lymphocytes responding to said donor antigens; and
(d) administering a preparation of hematopoietic stem cells from said non-syngeneic donor to said host mammal, wherein when said hematopoietic stem cells are stem cells obtained from the liver or yolk sac of embryos, then said embryos are non-human animal embryos.

### Brief Description of the Drawings

**Figure 1.** Effect of T cell depletion from a second BMC infusion on survival of tolerant mice.
**Figure 2.** GVHD-free survival (upper panel) and donor-type skin allograft survival (lower panel) of BALB/ c mice irradiated with various number of daily TLI fractions.
**Figure 3.** Xenograft survival of Lewis rat skin in B6 mouse recipients following a non-myeloablative tolerogenic protocol based on the use of sTLI, donor BMC and Cy.

### Detailed Description

The invention provides a new method for treating a host mammal to induce transplantation tolerance to cell, tissue and organ allografts and xenografts. Such transplants can provide replacement therapy for enzyme or metabolic disorders and adoptive immunotherapy for cancer and life-threatening infections in humans. The method also can be used to provide new animal models for tolerance induction toward allogeneic and xenogeneic cells.

In general, the method of treating host mammal includes (a) administering donor antigens from a non-syngeneic donor to the host mammal; (b) administering a non-myeloablative dose of lymphocytotoxic agent (e.g., cyclophosphamide) or tolerizing agent to the host mammal to selectively eliminate the host mammal's lymphocytes responding to the donor antigens; and (c) administering a preparation of hematopoietic stem cells from the non-syngeneic donor to the host mammal, wherein when said hematopoietic stem cells are stem cells obtained from the liver or yolk sac of embryos, then said embryos are non-human animal embryos.

Prior to step (a), the host mammal can be administered an immunosuppressive agent in a non-myeloablative regimen sufficient to decrease the host mammal's functional T lymphocyte population. The immunosuppressive agent can include one or more of an immunosuppressive drug, an alkylating agent, ionizing radiation, or anti-leukocyte or anti-leukocyte function antibodies. It is particularly advantageous to use a short course of TLI (sTLI) as the immunosuppressive agent, for example 1-12, frequently 1-6, doses of 200 cGy/dose.

The donor antigens administered to the host mammal can include non-cellular antigens, cells, tissues and/or organs. For example, the donor antigens can include hematopoietic stem cells or other viable cells. If the donor antigens include viable cells such as hematopoietic stem cells, then the immunosuppressive regimen referenced above should decrease the T lymphocyte population of the host to a level permitting at least transient survival of the donor's cells. For example the T lymphocyte population of the host can be decreased by 90%, 95% or 99%

The host mammal can be an animal or a human, for example a human cancer patient. The donor can be allogeneic or xenogeneic to the host mammal. Following performance of the method, the host mammal's blood can contain 20% or more donor cells. After administering the preparation of donor hematopoietic stem cells, with resultant engraftment of such cells in the host, the host can be treated with allogeneic cell therapy. This involves infusing allogeneic lymphocytes from the donor into the host mammal. Alternatively, the host can receive transplanted cells, tissues or organs from the donor, with the transplants becoming engrafted in the host due to the donor-specific tolerance induced in the host mammal.

In another aspect, the invention features a host-derived hematopoietic cell composition, including host-originating and donor-originating hematopoietic cells, with the composition being depleted of donor-specific, host- originating lymphocytes. The hematopoietic cell composition can be made by treating a host mammal as described above, then isolating the hematopoietic cell composition from the host mammal. Such hematopoietic stem cells are not fetal stem cells obtained from the liver or yolk sac of human embryos.

In a further aspect, the invention provides a method of making a non-human mammal/human chimera. This involves performing the methods described above, with the host mammal being a non-human mammal and the donor being a human being. The host mammal can be, for example, a rodent or pig. The result is a rodent, pig or other non-human mammal stably engrafted with human hematopoietic stem cells. Such hematopoietic stem cells are not fetal stem cells obtained from the liver or yolk sac of human embryos. As such, the non-human mammal host constitutes a hematopoietic mixed chimera.

The term "non-myeloablative" as used herein includes any therapy that does not eliminate substantially all hematopoietic cells of host origin. "Transplantation" as used herein refers to transplantation of any donor-derived material including cells, tissues and organs. The cells may be hematopoietic or non-hematopoietic. "Donor antigens" as used herein refers to any donor-derived material that elicits a host immune response, including non-cellular antigens, cells, tissues or organs. Stem cells are particularly useful as donor antigens. A "lymphocytotoxic agent" is an agent that kills T cells or paralyzes T cell function. A "tolerizing agent" is an agent that anergizes or "vetos" T cells by preventing development of normal T cell-dependent responses. The term "cancer" as used herein includes all pathological conditions involving malignant cells; this can include "solid" tumors arising in solid tissues or organs as well as hematopoietic tumors such as leukemias and lymphomas. The term "donor-specific tolerance" as used herein refers to tolerance of the host to donor-derived material.

Induction of donor-specific tolerance across strong major histocompatibility complex (MHC) and minor histocompatibility complex (MiHC) barriers, as well as across species barriers (xenogeneic tolerance) may be achieved in mammalian hosts using the tolerogenic treatment described herein. Induction of donor-specific transplantation tolerance while avoiding the need for maintenance immunosuppressive treatment is a highly desirable goal in clinical transplantation.

The non-myeloablative tolerogenic treatment described herein induces a state of long-lasting donor-specific tolerance to a wide variety of donor-derived material. Such an approach is attractive for allogeneic and xenogeneic transplantation of cells, tissues and organs in clinical settings, since all the steps of the protocol are well tolerated and relatively safe. Since there is no need to eradicate the entire host immunohematopoietic system during the course of the procedure, the recipients retain immune memory and are in a better position to resist graft-versus-host disease on the one hand and infectious complications on the other. This can be of crucial importance in clinical practice. The protocols for inducing donor-specific tolerance may be delivered, at least in part, as outpatient procedures.

Unless otherwise defined, all technical and scientific terms used herein have the same meaning as commonly understood by one of ordinary skill in the art to which this invention belongs. Although methods and materials similar or equivalent to those described herein can be used in the practice or testing of the present. invention, suitable methods and materials are described below. In case of conflict, the patent specification, including definitions, will control. In addition, the materials, methods, and examples are illustrative only and not intended to be limiting.

Other features and advantages of the invention will be apparent from the following further detailed description, and from the claims.

The present inventor has employed novel, non-myeloablative tolerogenic protocols to induce stable and donor-specific tolerance to non-syngeneic transplants (i.e., transplants of cells, tissues or organs not genetically identical to the host). A protocol for the tolerogenic treatment can be summarized as follows:
Step 1: Administer an immunosuppressive agent to a host mammal in a non-myeloablative regimen sufficient to decrease, but not eliminate, the host mammal's functional T lymphocyte population.
Step 2: Infuse donor antigens, preferably viable hematopoietic cells, from a non-syngeneic donor into the host mammal.
Step 3: Eliminate those host T lymphocytes responding to the infused donor antigens using a non-myeloablative dose of lymphocytotoxic or tolerizing agent.
Step 4: Administer a preparation of donor hematopoietic stem cells to the host mammal, wherein when said hematopoietic stem cells are stem cells obtained from the liver or yolk sac of embryos, then said embryos are non-human animal embryos.

This non-myeloablative, donor-specific tolerogenic treatment results in conversion of a host to a hematopoietic mixed chimera with high levels of donor hematopoietic cells. Typically, the mammalian hosts are human patients, although a recipient of the tolerogenic treatment may be any mammal. Non-syngeneic transplantation can include allogeneic as well as xenogeneic transplantation of organs, tissues or cells. Hence, hematopoietic stem cells and other donor antigens used in steps 2 and 4 may be derived from allogeneic or xenogeneic sources.

Human patients for which the tolerogenic treatment is appropriate include without limitation those with loss of organ or tissue function including loss of metabolic function such as in diabetes; patients with enzyme deficiencies caused by inborn genetic diseases such as Gaucher's disease, metachromatic leukodystrophy and Hurler's Syndrome; patients with autoimmune disorders such as lupus erythematosus and rheumatoid arthritis; and cancer patients. Patients suffering from heart, liver or kidney failure, for example, are excellent candidates for conditioning with the tolerogenic treatment prior to transplantation with the appropriate organ. Patients requiring a skin or bone graft may also be subjected to the tolerogenic treatment prior to grafting. Cancer patients receiving the tolerogenic treatment can include patients suffering from any malignancy, either solid tumors such as breast cancer or hematopoietic malignancies including acute and chronic leukemia, lymphoma, and myelodysplastic and myeloproliferative disorders.

Completion of the tolerogenic treatment protocols provides a platform for subsequent allogeneic cell therapy with donor lymphocyte infusions in cancer patients and in other patients with malignant and non-malignant diseases requiring bone marrow transplantation, since donor cells accepted by a tolerant host may induce graft-versus-leukemia (GVL) or graft-versus-tumor (GVT) effects. Such non-malignant diseases include without limitation aplastic anemia, genetic diseases resulting in enzyme deficiencies, and diseases caused by deficiencies in well-defined products of hematopoietic stem cells, such as osteoclast deficiency in infantile osteopetrosis and deficiencies in B cells and T cells in congenital and acquired immune-deficiency syndromes. Allogeneic cell therapy is described, for example, in PCT publication no's. WO 95/24910 and WO 96/37208.

In allogeneic cell therapy, an anti-tumor or other anti-host hematopoietic cell effect is achieved by administering allogeneic peripheral blood lymphocytes to the host, either alone or in combination with a T cell activator. Alternatively, allogeneic peripheral blood lymphocytes are "pre-activated" in vitro by a T cell activator such as interleukin-2 (IL-2) and then administered either alone or in combination with the same or different T cell activator. Preferably, one or more infusions of about 10⁵ to about 10⁹ cells/kg of allogeneic peripheral blood lymphocytes, including well-defined lymphocyte subsets, are administered. When preceded by the tolerogenic treatment described herein, these infusions of allogeneic lymphocytes are carried out with a much reduced chance of rejection of the anti-cancer effector cells, which need to become engrafted in the host. In addition, the risk of GVHD is reduced or eliminated by residual hematopoietic cells of the host and, if necessary, relatively late infusion of donor lymphocytes.

Allogeneic cell therapy following the tolerogenic treatment protocols described herein can be valuable not only in the context of cancer and other diseases, but also when it is desired to adoptively transfer immunity to infectious agents from the donor to the host. Thus, if a donor used in the tolerogenic protocols described herein is immune to an infectious agent (e.g., hepatitis B; see Ilan et al., Hepatology 18: 246-52 (1993)), this immunity can be transferred to a host by infusing lymphocytes from the donor to the host following completion of the tolerogenic protocols. Alternatively, the stem cell preparation infused in Step 4 of the tolerogenic protocols can itself provide the adoptive transfer of immunity, since stem cell preparations may contain immunocompetent lymphocytes.

In accordance with this invention, a significant number of the host mammal's functional T lymphocyte population remains in the host after the non-myeloablative regimen of Step 1. Nevertheless, engraftment of donor cells can occur because (a) donor-reactive host T lymphocytes are eliminated in step 3, and (b) donor-derived T lymphocytes and/or stem cells present in the subsequent infusion or infusions (Step 4) may act as "veto" cells to produce a veto effect. Veto cells, as used herein, include T lymphocytes, especially CD8⁺ T cells, that result in down regulation, rather than stimulation, of other T lymphocytes. Veto effects may be induced by other proliferating hematopoietic cells including T cell-depleted stem cells that are poorly immunogenic but that can veto host T cells. In the veto effect, host-originating T lymphocytes are down-regulated by donor-derived veto cells, including stem cells and/or lymphocytes. Other replicating donor-derived cells, or even non-cellular antigens, can also veto host allo- or xeno-reactive T cells if provided repeatedly and in relatively high concentrations. Similarly, immunocompetent T cells present in the donor infusion may be down-regulated by veto cells of host origin. Thus, tolerance of graft vs host and host vs graft may occur simultaneously due to a balanced equilibrium between veto cells of host and donor origin on the one hand and the degree of immunogenicity and alloreactivity of the graft on the other.

Examples of immunosuppressive agents useful in Step 1 include without limitation immunosuppressive drugs such as methotrexate and fludarabine (FLU); alkylating agents such as Cy, melphalan, thiotepa and busulfan; polyclonal and monoclonal anti-thymocyte globulin (ATG) and anti-lymphocyte globulin (ALG); and ionizing radiation such as TLI and TBI. Due to its non-selective effects on all of the host's hematopoietic cells and its severe immediate and long-term side effects, TBI is not preferred. If TBI is used, it should be at a dose level that causes no severe or irreversible pancytopenia. The non-myeloablative regimen advantageously is a short and well-tolerated course of TLI (sTLI) which may cause a major reduction in the number and/or function of host T lymphocytes in all lymphoid organs. As discussed below, it has been discovered that sTLI can effectively induce unresponsiveness to donor antigens at relatively low cumulative radiation doses.

The sTLI immunosuppressive regimen may comprise, for example, 1 to 12 daily fractions of 200 cGy/each depending on the host-versus-graft potential and the T lymphocyte content in the stem cell preparation administered in Step 4. Stem cell preparations rich in T lymphocytes may require only 1-3 sTLI fractions, or may not require immunosuppression at all (zero sTLI fractions). Transplantation of T cell-depleted stem cell preparations or stem cell preparations with low levels of T lymphocytes, however, may require the use of 4-12 fractions. The sTLI regimen causes only a transient reduction in the number of host T lymphocytes and is clinically feasible on an outpatient basis. There are no anticipated severe side effects since a routine cumulative dose of TLI used clinically for lymphoma patients consists of 4,400 cGy.

Preferably, the immunosuppressive agent transiently decreases the host functional T lymphocyte population by at least about 90%. More preferably, the non-myeloablative regimen transiently decreases the host functional T lymphocyte population by at least about 95%, and most preferably, by at least about 99%. Reductions of less than 90% of the lymphocytes are also within the scope of this invention, provided that transient survival of donor antigens, provided in Step 2, is possible.

In some donor/recipient combinations, tolerance to donor antigens may be inducible without the necessity of performing Step 1. In this case, the preparation of donor hematopoietic stem cells administered in Step 4 must contain a sufficient number of T cells to provide a protective veto function against residual host T cells escaping the effects of Step 3.

In Step 2 of the tolerogenic treatment, antigens from a non-syngeneic donor are administered to the host mammal in order to stimulate and cause proliferation of donor-specific T lymphocytes of the host. The stimulated sub-population of donor-specific host T lymphocytes is then eliminated or tolerized in Step 3. The donor antigens may be administered (Step 2) to the host after the non-myeloablative immunosuppressive regimen (Step 1) described above. Alternatively, the donor antigens may be administered to a non-immunosuppressed host (if Step 1 is excluded as described above).

The donor antigens administered in step 2 can include, without limitation, non-cellular antigens, cells, organs, tissues or tissue extracts, or even anti-idiotypic antibodies that mimic donor antigens. In general, any donor antigens that elicit an immune response in the host are within the scope of this invention. Any source of donor antigens from a non-syngeneic donor can be used, and the non-syngeneic donor can be allogeneic or xenogeneic to the host.

The infusion of donor antigens should comprise donor antigenic determinants for which tolerance is desired. For example, if it is desired to transplant into the host donor-derived material bearing only class I histocompatibility antigens, it may be necessary to eliminate only class I-reactive host T lymphocytes in Step 3. This could be accomplished by infusing, in Step 2, donor antigens bearing only class I antigenic determinants. On the other hand, additional donor antigenic determinants may be present in the infusion of Step 2 even though host tolerance to these additional antigenic determinants may not be necessary. Thus, elimination of class I- and class II-reactive host T lymphocytes by infusion of donor antigens bearing class I and class II antigenic determinants may be performed even if the later transplanted donor material bears only Class I antigenic determinants.

The donor antigens infused in Step 2 can be viable hematopoietic stem cells from a non-syngeneic donor. The donor hematopoietic stem cells generally are not T cell depleted, although use of T cell depleted donor hematopoietic stem cells in Step 2 is also within the scope of this invention. Donor hematopoietic stem cells for use in Steps 2 and/or 4 may be obtained, for example, by direct extraction from the bone marrow or from the peripheral circulation following mobilization from the bone marrow. The latter can be accomplished by treatment of the donor with granulocyte colony stimulating factor (G-CSF) or other appropriate factors that induce mobilization of stem cells from the bone marrow into the peripheral circulation. The mobilized stem cells can be collected from peripheral blood by any appropriate cell pheresis technique, for example through use of a commercially available blood collection device as exemplified by the CS3000 Plus blood cell collection device marketed by the Fenwal Division of Baxter Healthcare Corporation. Methods for performing apheresis with the CS 3000 Plus machine are described in Williams et al., Bone Marrow Transplantation 5: 129-133 (1990) and Hillyer et al., Transfusion 33: 316-321 (1993). Alternative sources of stem cells include neonatal stem cells (e.g., cord blood stem cells) and fetal stem cells (e.g., fetal liver of yolk sac cells). Stem cells that have been expanded in vitro with a mixture of hematopoietic cytokines also may be used. Other useful stem cell preparations include stem cells that have been transduced with genes encoding donor-type MHC class I or class II molecules, as well as stem cell preparations containing stem cells and/or T cells transduced with herpes simplex thymidine kinase or other "suicide" genes to render the mature T cells sensitive to ganciclovir or other appropriate drugs in the event of severe GVHD.

With respect to Step 3, "elimination" of the proliferating donor-specific host T lymphocytes as used herein includes host T lymphocyte inactivation or tolerization as well as host T lymphocyte death. Examples of lymphocytotoxic agents useful in Step 3 include Cy, melphalan and methotrexate. Cy, for example, is a short acting cytotoxic drug known for its ability to kill lymphocytes, especially cells that proliferate in response to antigenic stimulation (Bach JF, Amsterdam: North-Holland (1975); Aisenberg et al., Nature, 213:498 (1967); Paul WE, Fundamental Immunology. New York: Raven, (1984)). Cy can also facilitate activation of antigen-specific T cell suppressors responsible for maintenance of the tolerant state. Chernyakhovskaya et al., Transplantation, 38:267 (1984); Maeda et al., Transplantation, 57:461 (1994). Other agents known to eliminate proliferating T cells in response to donor antigenic stimulation may also be used, including monoclonal antibodies against activation markers of T lymphocytes such as anti-CD25, anti-DC69 and anti-Ia/DR antibodies. Alloreactive host T cells may be tolerized, rather than killed, by using agents that block co-stimulation in conjunction with activation, since T cell engagement with antigen without a second signal provided by co-stimulation results in tolerance. Such tolerizing agents include without limitation CTLA4-Ig, anti-B7.1 or anti-B7.2, anti-CD28, and antibodies against adhesion molecules such as anti-LFA1, anti-CD44 and similar agents. If tolerizing agents are used, steps 2 and 3 can be performed simultaneously.

In order to ensure an acceptable state of stable, mixed chimerism with relatively high numbers of circulating donor cells, donor hematopoietic stem cells are administered to the host following performance of Step 3. This infusion of donor stem cells (Step 4) is derived from the same donor, or from a donor genetically identical to that providing the antigens for Step 2. Hematopoietic stem cells from bone marrow, from mobilized peripheral blood populations, or other stem cell preparations as described above, may be used. The number of stem cells administered in Step 4 can vary depending on the T cell content of the stem cell preparation. If the preparation is not T cell-depleted, then relatively small numbers of stem cells generally are administered. If the stem cell preparation is T cell-depleted, then larger numbers of stem cells can be administered since there is no risk of GVHD.

The donor hematopoietic stem cells of the second infusion may or may not be T cell depleted, depending on the immunologic disparity between the donor and recipient, the intensity of immunosuppression given in Step 1 and the degree of chimerism desirable in view of the immunogenicity of the graft. When higher fractions of sTLI (4-12), or other immunosuppressive agents providing equivalent immunosuppression, are used in the immunosuppressive regimen of Step 1, the second infusion comprising donor hematopoietic stem cells typically is T cell depleted to control for GVHD. When Step 1 involves little immunosuppression (for example, 1-3 fractions of sTLI), or when Step 1 is eliminated altogether, the infusion of donor hematopoietic stem cells in Step 4 typically is not T cell depleted. If not T cell depleted, the donor stem cells provided in Step 4 can be infused in graded increments over a period of weeks or several months, while monitoring for signs of GVHD.

In mouse experiments reported below, the mice received sTLI of 0-6 fractions of 200 cGy/fraction (Step 1). The donor-reactive T cells of the host were activated (Step 2) by injecting non-T cell depleted donor BMC (3x10⁷ cells). The activated host T cells were subsequently eliminated (Step 3) by a non-myeloablative dose (200 mg/kg or 3 doses of 60 mg/kg) of Cy. Mixed chimeras with low levels (e.g., 7%-20%) of donor cells in the blood were predominant after the Cy treatment. Induction of higher levels of hematopoietic mixed chimerism (e.g., >20% of donor cells in blood) was achieved by administering (Step 4) a second infusion comprising donor hematopoietic stem cells, allowing life-long survival of donor skin allografts.

In mice treated with 6 doses of TLI, Step 4 was required to achieve a level of tolerance permitting acceptance of full thickness skin allografts. It is well known that full-thickness skin presents the most stringent test for donor-specific tolerance. Skin allograft acceptance can be accomplished only in stable chimeras (Maeda et al., J. Immunol., 150:753 (1993)) and success of skin acceptance may depend on the level of donor-derived cells in the host's blood.

In further experiments reported below, deletion of all host-derived, donor-reactive T lymphocytes following Step 3 permitted rapid engraftment of even low numbers of donor stem cells (2-3 x 10⁶/mouse) administered in Step 4, which normally would not be sufficient for induction of stable mixed chimerism. In parallel with this, the full anti-donor unresponsiveness induced following Step 3 also resulted in exquisite sensitivity to donor T lymphocytes, leading to lethal GVHD. Hence, whenever donor-reactive host T cells are effectively depleted, elimination of immunocompetent T lymphocytes from the hematopoietic stem cell preparation administered in Step 4, or use of lifespan-limited lymphocytes (e.g., carrying suicide genes), is crucial for prevention of GVHD. Due to the selective deletion of donor-reactive host lymphocytes, even 2-3x10⁶ BMC (T cell-depleted), administered following Step 3, engrafted and converted host mice into stable mixed chimeras with relatively high levels (20% - 50%) of donor-derived hematopoietic cells in the blood.

T cell depletion of donor stem cell preparations has been known to increase the risk of graft rejection. Thus, inoculation with extremely large numbers of donor stem cells has been mandatory for engraftment of T cell depleted BMC, especially in recipients conditioned with previous non-myeloablative protocols. Truitt et al., Blood 77, 2515-2523 (1991); Reisner et al., Immunol. Today 16, 437-440 (1995); Bachar-Lustig et al., Nat. Med. 12, 1268-1273 (1995). The ability to induce engraftment using low numbers of donor hematopoietic stem cells (T lymphocyte depleted) is a significant advantage of the present protocols. This is due to the improved acceptance of donor hematopoietic stem cells on the one hand combined with a reduced risk of GVHD that would otherwise follow from the use of higher stem cell inocula, on the other. Notably, animals non-specifically immunosuppressed by sTLI and Cy (without infusion of donor-antigens prior to Cy) were shown to reject T lymphocyte depleted BMC. Thus, the present data clearly show the advantage of donor-specific tolerogenic conditioning in comparison with non-specific immunosuppression approaches, while avoiding potentially hazardous high doses of TBI.

Interestingly, life-long tolerance to full thickness donor-derived skin grafts was also accomplished in recipients who were not subjected to Step 1 and in recipients in which Step 1 involved only a single fraction of TLI (200 cGy). Thus, a balance exists between the intensity of the conditioning of the host and the susceptibility of the host to GVHD induced by the presence of donor-derived T cells: recipients of a single fraction of sTLI could resist GVHD induction by a large inoculum of non-T cell depleted donor BMC whereas the sensitivity of the hosts to GVHD was increased in recipients conditioned with 6 fractions of sTLI. Hence, a second infusion (Step 4) comprising non-T cell depleted donor BMC could be used relatively safely in recipients of 1 dose of sTLI whereas T cell depletion of the second infusion was mandatory in recipients of 6 fractions of sTLI. The sensitivity of the 6 x sTLI recipients to develop GVHD was likely due to inability to veto the host-reactive donor cells due to effective depletion of all host T cells.

Tolerant mixed hematopoietic chimeras generated by the tolerogenic treatment described herein remain immunocompetent to third party grafts. In experiments described below, all tolerant B6→BALB/c chimeras that accepted B6 skin allografts rejected non-relevant CBA skin grafts within 16-20 days (n = 11). Thus, tolerance induction neither eliminated nor impaired normal reactivity by the host immune system retained in the mixed chimera. This is an important advantage of the method, since recipients are not immunocompromised due to transient loss of all host-derived immune cells, which is otherwise unavoidable when chimeras are comprised of 100% donor cells following TBI. A patient who retains a host-derived immune apparatus with memory cells is in a better position to resist primary and secondary infections. This retained resistance to intercurrent infections, particularly to viral agents infecting host target cells, is of crucial importance. This is because the donor hematopoietic cells may be MHC disparate and, therefore, incapable of providing immune protection against virally-infected host tissues.

The above-described tolerogenic treatment may be employed to induce transplantation tolerance across xenogeneic barriers. Xenogeneic skin transplantation may be considered the most stringent test for donor-specific tolerance. As described below, the present inventor has succeeded in inducing permanent tolerance in mouse-to-rat skin grafts. The same donor-specific tolerance induction protocol presented herewith can be applied to xenogeneic transplantation in humans. The xenogeneic graft (e.g., pancreatic islets) may be taken from non-human mammals and transplanted into humans.

A tolerogenic treatment for xenogeneic transplantation may be performed as follows. sTLI (Step 1) is carried out, followed by an infusion (Step 2) of xenogeneic donor antigens, for example, BMC. Subsequently, at least one non-myeloablative dose of lymphocytotoxic or tolerizing agent is administered (Step 3). If necessary, the lymphocytotoxic agent can be administered in multiple low doses over several days. Administration of the lymphocytotoxic agent is followed by a infusion of a preparation comprising T cell-depleted donor hematopoietic stem cells (Step 4). The stem cells may be obtained from the blood or bone marrow of an adult donor. Alternatively, partially immunocompetent cord blood cells may be used, or even fetal stem cells obtained from the liver or yolk sac of embryos provided that said embryos are non-human animal embryos. Stem cells that have been expanded in vitro with a mixture of hematopoietic cytokines also may be used. Administration of stem cells in Step 4 leads to engraftment of the xenogeneic donor stem cells and permanent transplantation tolerance of the host to donor derived organs. In an alternative embodiment, xenogeneic transplantation may be performed without administration of a non-myeloablative regimen (Step 1 eliminated) and with the second infusion (Step 4) comprising non T cell depleted donor hematopoietic stem cells.

In another embodiment, the invention provides a method of making a non-human mammal/human hematopoietic chimera. The method comprises making a non-human mammal tolerant to antigens originating from a human donor, using the non-myeloablative tolerogenic treatment described herein. That is, the non-human mammal functions as the "host mammal" in the protocols described above, and a human being is the "donor." For exatple, a rodent can be tolerized to human cells, tissues and organs by employing Steps 1-4 of the disclosed tolerogenic protocol to produce a mixed chimera rodent permanently engrafted with human hematopoietic cells. It is known that such hematopoietic engraftment is possible even between disparate species. For example, it has been demonstrated that human hematopoietic cells can engraft in mice. See, for example, Marcus et al., Blood 86: 398-406 (1995). In those cases where survival and functioning of human hematopoietic cells is less than optimal in non-human mammalian hosts, it is possible to provide the host mammal with human hematopoietic cytokines in order to ensure engraftment of the human cells.

There are numerous uses for such chimeric animals. For example, since the host mammals have been tolerized to the human donor, it is possible for human tissues, e.g., tumors or HIV-infected hematopoietic cells, to be transplanted into and accepted by these rodents in order to produce rodent models of human disease. Thus, these non-human mammal/human chimeras may be used to study biological phenomena related to human disease, including testing of new drugs.

Production of non-human mammal/human hematopoietic mixed chimeras is of even greater significance for those non-human mammalian species targeted as potential sources of cells, tissues and organs for transplantation into human patients. For example, it is widely recognized that pigs are a potential useful source of tissues and organs for transplantation into humans. Such porcine materials are subject to an immediate, "hyperacute" rejection response when transplanted into human patients, as well as to longer-term immune-mediated rejection by the human host. Pigs are being genetically engineered or otherwise treated to protect tissues and organs of such pigs from being hyperacutely rejected when transplanted into a human patient. This can be accomplished, for example, by providing the pigs human genes encoding human complement regulatory proteins, or by "knocking out" the genes responsible for production of pig antigens recognized by preformed xenoantibodies present in all humans. See, for example, PCT/US96/15255 and PCT/IB95/00088.

A "two-way" variation of the present tolerogenic protocols can be applied to such genetically engineered pigs as well as to other donor mammals to allow for ready transplantation of xenogeneic donor cells, tissues and organs into humans. For example, in a preliminary tolerization procedure, a human patient can function as an initial "donor" to provide antigens and hematopoietic stem cells to a "host" pig in the 4-Step protocol described above. As a result, the pig is transformed into a pig/human hematopoietic mixed chimera, with the pig's hematopoietic cells being tolerized to the human patient's cells, tissues and organs. Following this, the roles of the human patient and pig are reversed, with the pig becoming the donor and the human patient becoming the host in the 4-Step protocol. That is, the pig's hematopoietic cells, with T cells tolerant of the human patient, may be used in the 4-Step protocol for transformation of the human patient into a human/pig hematopoietic mixed chimera. The human patient is then able to accept cells, tissues and organs from the pig, for the reasons discussed above. The crucial advantage is that all of this can be accomplished while avoiding the risk of xenogeneic GVHD engendered by immunocompetent T cells of the pig, since the pig's T cells were made tolerant to the patient in the preliminary tolerization procedure. Thus, assuming the hyperacute rejection response can be overcome in other ways (e.g., genetic engineering of the animal providing the transplanted material), the present invention allows for xenogeneic transplantation of cells, tissues and organs into humans without the need for long-term immunosuppression.

Another aspect of the invention is a hematopoietic cell composition derived from a host treated with the donor-specific tolerogenic treatment described above. Such hematopoietic stem cells are not fetal stem cells obtained from the liver or yolk sac of human embryos. The cell composition comprises host-originating lymphocytes and donor-originating lymphocytes. The proportion of donor-originating lymphocytes may vary. Preferably, the donor-originating lymphocytes comprise about 5% to about 50% of the lymphocyte composition. Most preferably, the donor-originating lymphocytes comprise about 20% to about 50% of the lymphocyte composition. The hematopoietic cell composition is specifically depleted of donor-specific, host-originating lymphocytes. "Depleted" in this context refers to reduction in numbers of T lymphocytes or reduction in T lymphocyte function sufficient to eliminate or significantly reduce anti-donor responses in the host and thus to reduce the risk of GVHD if the composition is administered to the donor. In a related aspect, the invention involves a method of making the above described hematopoietic cell composition. The method includes subjecting a host mammal to the tolerogenic treatment described above using an. allogeneic or xenogeneic donor. After tolerogenic treatment, the method involves isolating a composition of hematopoietic cells from the host. This cell composition contains host and donor-originating hematopoietic cells, but is depleted of donor-specific host-derived T lymphocytes.

The invention will be further understood with reference to the following illustrative embodiments, which are purely exemplary, and should not be taken as limiting the true scope of the present invention as described in the claims.

### EXAMPLE 1

### Materials and Methods Used in Examples 2-9

### Animals

Inbred BALB/c (H-2^{d}), C57BL/6 (B6) (H-2^{b}), DBA, CBA (H-2^{k}) mice and Lewis rats were purchased from the Hebrew University Hadassah Medical School Animal Facility in Jerusalem, Israel, with breeding pairs originating from Harlan-Olack, Bicester, UK. Two to three month-old mice were used for the study. Mice were kept under standard conditions with food and water provided ad lib. Most of the experiments were carried out in B6 → BALB/c strain combination.

### TLI

Mice were anesthetized and then positioned in an apparatus designed to expose the major lymph nodes, thymus, and spleen to ionizing irradiation, while shielding most of the skull, ribs, lungs, hind limbs and tail with lead, as previously described. Slavin et al., J. Exp. Med., 146:34 (1977). Radiation was delivered by a Phillips X -ray unit (250 kv, 20 mA) at a rate of 70 cGy/min, using a Cu 0.2-mm filter. The source-to-skin distance was 40 cm.

### Tolerogenic Treatment

The basic protocol for conditioning prior to transplantation included sTLI (1-6 daily exposures of 200 cGy) to a total dose of up to 1,200 cGy, followed by intravenous inoculation with 3×10⁷ BMC on the day after the last TLI dose. Some mice (see Example 9) were not administered any sTLI. One day after BMC infusion, experimental mice were injected with 200 mg/kg Cy (Taro, Israel) intraperitoneally. Cy was freshly dissolved in sterile phosphate-buffered saline prior to injection. Modifications of the Cy protocol to induce tolerance to xenografts are described in Example 8. A second infusion of donor BMC, after Cy, was also administered in some of the mice.

### Preparation of Bone Marrow and Spleen Cells

Single cell suspensions of BMC and spleen cells were prepared in PBS or RPMI 1640 medium supplemented with 100 µg/ml streptomycin and 100U/ml penicillin (Biological Industries, Beit Haemek, Israel). BMC were infused into the lateral tail vein in a total volume of 0.5 ml.

### Preparation of Blood Cells for Infusion

Pooled fresh blood was collected into heparin-containing tubes (preservative-free). Each recipient was infused with 0.5 ml into the lateral tail vein.

### T cell Depletion of BMC With Monoclonal Antibodies

Monoclonal rat anti-mouse Thy1 antibodies (YTS 148.3, IgM and YTS 154.7, IgG2b) were obtained from Dr. H. Waldmann (Oxford University, UK). BMC (10⁷/ml) were incubated with YTS 148.3 antibody at a final dilution of 1:200 for 40 min, washed and incubated with Low-Tox rabbit complement (Cedarlane, Canada) at a final dilution of 1:10 for an additional 60 min at 37°C, washed and injected intravenously into recipients. YTS 154.7 antibody was used for depletion of T cells from BMC in vivo; BMC (3×10⁶/ml) were incubated with 750 µg, 150 µg or 30µg of the antibody for 60 min at 4°C and the mixture was injected intravenously into recipients.

### Skin Grafting

Skin grafting was carried out 20 days after completion of the tolerogenic treatment. A full-thickness skin graft measuring 1cm × 1cm was adjusted to the graft bed by 4 Thomas surgery clips (Thomas Scientific, USA ). The panniculus carnosus was kept intact in the graft bed. The graft was considered to be accepted when hair of donor color grew on the soft flexible underlying skin, and rejected when donor epithelium was lost.

### Implantation of Bone Marrow Plugs

The femora of B6 mice were freed of muscle and irradiated with 400 cGy in vitro to eliminate most of the hematopoietic cells. Marrow plugs were mechanically pressed out of the femur canal with a mandrin and 2 plugs were implanted under the left kidney capsule of each recipient, as described in Chertkov et al., Rad. Res. 79, 177-186 (1979).

### Heterotopic Heart Grafting

Hearts of 1-2 day old B6 mice were transplanted into the ear skin pocket 20 days after tolerogenic treatment, according to the methods of Chernyakhovskaya et al., Transplantation, 29:409 (1980). An ECG was first recorded two weeks after grafting and thereafter at weekly intervals.

### Assay for Chimeras

The percentage of donor-type cells was assayed in the blood of recipients, using a standard complement-dependent microcytotoxicity test. Morecki et al., J. Exp. Med., 165:1468 (1987). Specific allo-antisera against H-2^{b} and H-2^{d} histocompatibility antigens were prepared by cross-immunization using skin grafting between BALB/c and B6 strains of mice, followed 2 and 4 weeks later by intraperitoneal injections with 5 × 10⁷ spleen cells.

### Polymerase Chain Reaction (PCR)

PCR was carried out on material derived from blood samples as described previously. Pugatsch et al., Leukemia Res. 17, 999-1002 (1993). Briefly, blood samples were lysed in distilled water and centrifuged at 12,000 x g. Supernatants were discarded and 50 µl of 0.05 M NaOH were added to the cell pellets. Samples were boiled for 10 min., then 6 µl of 1 M Tris (pH 7.2) were added. Samples were then centrifuged at 12,000 x g and supernatants were used for assay. The 5'- and 3'-oligonucleotide primers chosen for amplification and the PCR reaction conditions are described in Pugatsch et al. Reaction products were visualized on 1.6% agarose gels (Sigma, USA) containing 0.05 µg/ml ethidium bromide.

### EXAMPLE 2

### Nonspecific immunosuppression of mice treated with sTLI alone or with sTLI and Cy

In the first set of experiments (Table 1), BALB/c mice were given 6, 8 or 12 (experimental groups) or 17 (control groups) fractions of TLI at 200 cGy/fraction. After TLI, 3 x 10⁷ BMC from B6 donors were administered one day after the last TLI. Skin allografts from B6 donors were transplanted 20 days after transfer of the BMC.

In a second set of experiments (Table 2), 5 groups of BALB/c recipients were administered sTLI of 6 fractions of 200 cGy/day, followed a day later with 200 mg/kg Cy intraperitoneally. After the above conditioning, the five groups were treated as described below. Group 1 received 3 x 10⁷ BMC, whereas group 2 received 3 x 10⁷ BMC and 5 x 10⁶ spleen cells. Group 3 received 0.3 ml of whole blood. Group 4 received 3 x 10⁶ BMC, whereas group 5 received 3 x 10⁶ T cell depleted BMC. T cell depletion was performed in vitro with monoclonal antibody Thy 1 and rabbit complement, as described in Example 1. For all of the above five groups, cells and whole blood were from B6 donors and were infused intravenously. Skin allografts were transplanted 20 days after BMC or blood transfer.

### RESULTS

A short course of TLI (sTLI), in contrast to a long course of TLI (17 fractions, 200cGy each), was insufficient for acceptance of stem cells from allogeneic BMC or blood. Table 1 shows that none of the BALB/c mice receiving 3×10⁷ fully mismatched BMC from B6 donors became hematopoietic cell chimeras after 6 fractions of TLI, while consistent acceptance of allogeneic BMC was obtained after 17 fractions of TLI. As shown in Table 1, after treatment with sTLI and allogeneic BMC or allogeneic blood cells, BALB/c recipients stayed alive, none developed GVHD and all rejected B6 skin allografts. Thus, after sTLI alone, sufficient numbers of immunocompetent cells remain in the host to reject a donor allograft.

**Table 1.**

| Incidence of allogeneic BMC and skin graft acceptance after fractionated total lymphoid irradiation. | | |
|---|---|---|
| No of TLI Fractions | % of donor cells in blood 100 days after cell transfer^{a} | Skin allograft survival >100 days^{c} |
| 6 | 0(10)^{b} | 0/10 |
| 8 | 0(3), 56(1) | 1/4 |
| 12 | 0(3), 50, 90 | 2/5 |
| 17 | 82, 85, 90(2), 93 | 5/5 |

| | | |
|---|---|---|
| ^{a}BMC (3x10⁷) from B6 donors were given to BALB/c recipients one day after the last TLI. | | |
| ^{b}Number of mice with the same level of chimerism is given in parentheses. | | |
| ^{c}Skin allografts from B6 donor were transplanted 20 days after cell transfer. | | |

**Table 2.**

| Skin allograft survival and GVHD related death after transplantation of allogeneic cells and skin graft following sTLI in combination with a single injection of Cy. | | | | |
|---|---|---|---|---|
| No | Donor Cells^{a} day 0 | GVHD related death | Mice survival Mean ± SD days | Skin graft survival^{c} >100 days |
| 1 | BM 3x10⁷ | 22/24 | 39 ± 8 | 2/2 |
| 2 | BM 3x10⁷ and spleen-5x10⁶ | 14/14 | 10 ± 3 | NT |
| 3 | Blood 0.3 ml | 7/7 | 30 ± 12 | NT |
| 4 | BM 3x10⁶ | 14/15 | 40 ± 7 | 1/1 |
| 5 | BM 3x10⁶ T cell depleted^{b} | 0/7 | >100 | 1/7 |

| | | | | |
|---|---|---|---|---|
| ^{a}Cells or whole blood from B6 donors were transferred intravenously to BALB/c recipients after sTLI and Cy. | | | | |
| ^{b}T cell depletion was performed in vitro with mAb anti Thy 1 and rabbit complement. | | | | |
| ^{c}Donor skin allografts were transplanted 20 days after cell transfer. NT - not tested. | | | | |

In sharp contrast, a single injection of Cy (200 mg/kg) given one day after the last fraction of sTLI (6 fractions, 200cGy each), increased the non-specific immunosuppression and allowed the hosts to accept BMC, spleen and blood cells. However, all recipients developed typical acute GVHD which was lethal in most cases (Table 2). The survival time of the recipients with GVHD appeared to be a function of the number of mature immunocompetent T cells present in the inoculum. Mean survival time of mice inoculated with 3x10⁷ BMC was four times longer as compared with recipients of an equal number of BMC mixed with 5×10⁶ B6 spleen cells (Table 2, groups 1 & 2). Transfer of fewer BMC (3×10⁶ instead of 3×10⁷) did not prolong survival significantly (Table 2, groups 1 & 4).

GVHD was successfully prevented in the mice of group 5 (Table 2), who received T cell depleted BMC after sTLI and Cy. Although none of these experimental mice developed GVHD and all of them remained alive, all but one rejected donor BMC. Accordingly, prolonged skin allograft survival was observed in only 1/7 recipients.

### EXAMPLE 3

### Antigen-specific elimination of residual donor-alloreactive host immunocompetent T cells

Three groups of BALB/c recipient mice were administered sTLI in 6 fractions of 200 cGy/day. Non T cell depleted BMC (day 0) from B6 donors were transferred intravenously to the BALB/c recipients in all three groups. The next day all of the recipients were administered 200 mg/kg of Cy. One group (Table 3, Group 1) received a skin graft from B6 donors at day 20. The second group (Table 3, Group 2) received 3 x 10⁷ non T cell depleted BMC from B6 donors on day 2 followed by a skin graft at day 20. The third group (Table 3, Group 3) received 3 x 10⁶ non T cell depleted BMC from B6 donors on day 2 followed by a skin graft at day 20.

Levels of donor cells in blood were assayed in all surviving mice at day 100 according to the protocol of Example 1.

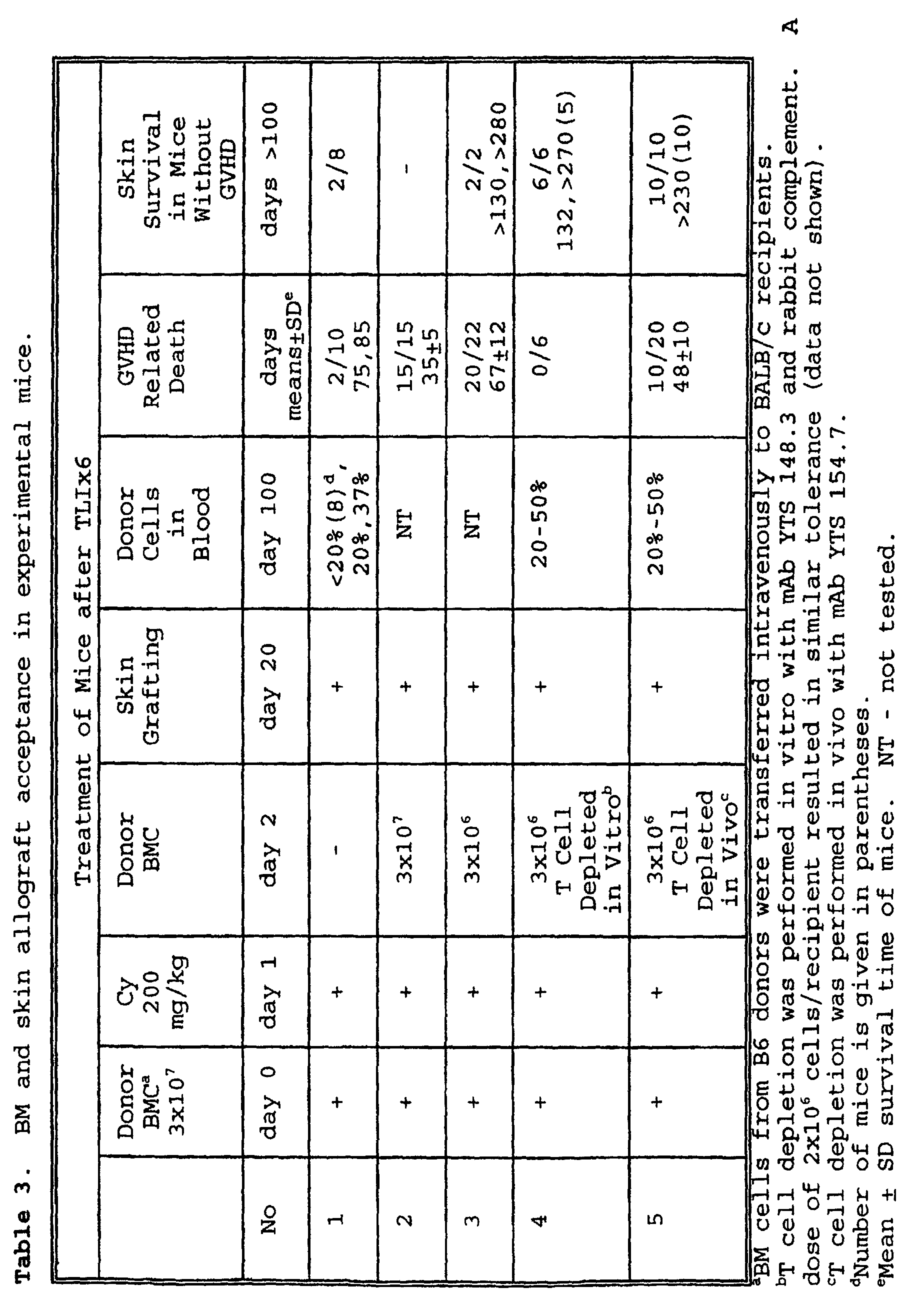

### RESULTS

Most of the mice converted to mixed chimeras with a relatively low number (7%-20%) of donor hematopoietic cells in the blood (Table 3, group 1). Donor non T cell depleted BMC transplanted one day after the Cy engrafted successfully but induced GVHD (Table 3, groups 2, 3).

### EXAMPLE 4

### Establishment of stable GVHD-free mixed chimeras by transfer of low dose T cell depleted donor BMC

Two groups of BALB/c recipient mice were treated as described in Example 3 except that T cell depleted BMC were administered on day 2 instead of non T cell depleted BMC. One group (Table 3, Group 4) was administered 3 x 10⁶ of in vitro T cell depleted BMC from B6 donors. A total of 2 x 10⁶ T cell-depleted BMC was sufficient, as demonstrated in one additional experiment (data not shown). The other group (Table 3, Group 5) was administered 3 x 10⁶ of in vivo T cell depleted BMC from B6 donors. T cell depletion was performed as described in Example 1.

### RESULTS

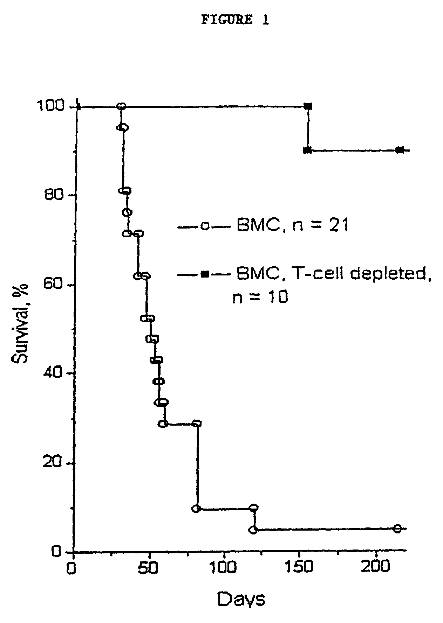

Elimination of immunocompetent T cells from allogeneic donor BMC was crucial for prevention of GVHD (Figure 1). In mildly immunosuppressed recipients, after in vitro depletion of T cells from donor BMC, all treated mice converted to stable mixed chimeras with 20%-50% of donor cells in the blood. The stable mixed chimeric mice accepted full-thickness B6 skin allografts and survived for 152-290 days without clinical signs of GVHD (Table 3, group 4). Similar results were obtained using an identical protocol for BALB/c → B6 chimeras with permanent (>150 days) survival of BALB/c skin allografts (data not shown).

Depletion of T cells in vivo from donor BMC was less successful than T cell depletion in vitro (Table 3, group 5). Of the mice that received in vivo T cell depleted BMC, only half remained free of GVHD and survived for >250 days (Table 3, group 5). These animals were all confirmed to be stable mixed chimeras and all accepted donor skin allografts.

### EXAMPLE 5

### Tolerance to allografts of donor BM stroma and neonatal heart

Five groups of BALB/c recipient mice were conditioned with sTLI by administration of 6 fractions of 200 cGy/day. All of these groups then received 3 x 10⁷ non T cell depleted BMC of B6 donors intravenously one day after the last TLI fraction. A dose of Cy (200 mg/kg) was given one day after the BMC transfer but before allograft transplantation. Twenty-four hours after the Cy, Group 1 (Table 4) was transplanted with non T cell depleted BMC whereas Group 2 (Table 4) was transplanted with in vitro T cell depleted BMC. Group 3 (Table 4) was transplanted with BMC stroma one day after Cy, Group 4 (Table 4) with heart 20 days after Cy and Group 5 with skin 20 days after Cy. All of the allografts were from B6 donors.

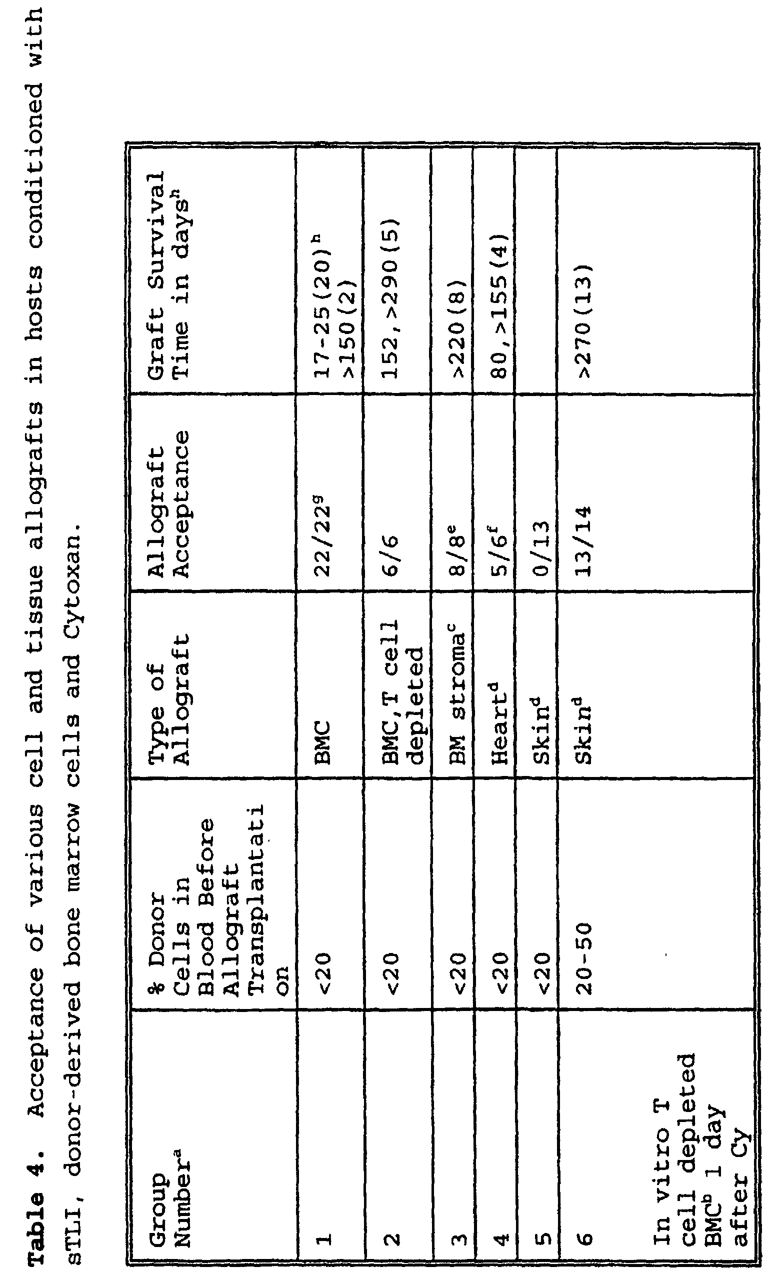

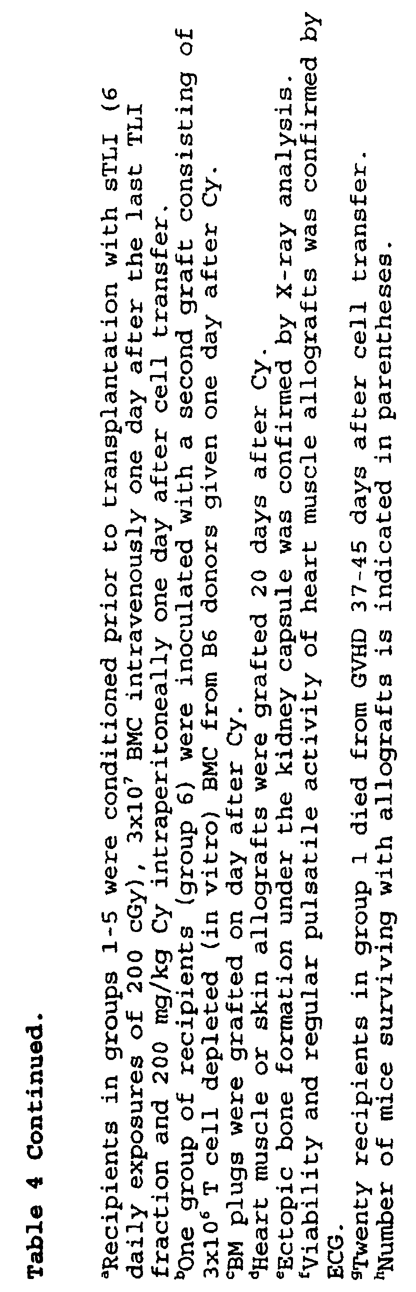

### RESULTS

Mice that were given a second infusion of unmanipulated donor BMC had graft acceptance but 20 of the 22 mice died from GVHD 37-45 days after cell transfer. Mice transplanted with T cell depleted BMC in the second infusion had graft acceptance and much higher graft survival.

Implantation of two femoral plugs from B6 donors under the kidney capsule of BALB/c recipients one day after Cy without a second inoculum of T cell depleted donor BMC resulted in formation of fully developed ectopic bone confirmed by X-ray analysis and subsequently by autopsy. This bone supported both donor and recipient hematopoiesis (Table 4, Group 3). Fragments of the ectopic osteo-hematopoietic site, when retransplanted under the kidney capsule of normal mice, formed bones and ectopic hematopoietic sites in secondary recipients of donor origin (9/9 successful allografts in B6 recipients) but not in BALB/c mice (0/9). These data indicate that ectopic osteo-hematopoietic sites in these mice were of donor origin.

The same treatment was also sufficient for acceptance of heterotopically transplanted neonatal heart grafts obtained from 1-2 day old B6 donors. Results show that the heart muscle transplanted into an ear skin pocket of BALB/c recipients 20 days after the tolerogenic treatment were ECG positive for > 80 days (Table 4, Group 4). In all mice that accepted donor-derived neonatal heart grafts, contractions of the heart muscle could also be detected visually. Mice that received only sTLI and Cy rejected both femoral plugs and neonatal heart grafts from B6 donors within 30 days (Data not shown).

Most of the recipients that received sTLI, BMC and Cy accepted donor BMC, BM stromal precursor cells and neonatal heart allografts. However, the conditioning was not sufficient to ensure survival of skin allografts obtained from the same donor (Tables 3 & 4).

### EXAMPLE 6

### Conditions required for stable donor skin allograft acceptance in mice

A group of mice were administered sTLI, BMC and Cy as described in Example 5. This group (Table 4, Group 6) also received a second inoculation of 3 x 10⁶ of B6 in vitro T cell depleted B6 donor BMC one day after Cy but prior to skin allograft.

### RESULTS

Mixed hematopoietic cell chimerism was documented among all experimental animals tested, those that accepted as well as those that rejected donor-type skin allograft. However, the level of chimerism was clearly higher in the mice that, after administration of Cy, received donor BMC in suspension or within a BM femoral plug (20%-50% donor cells) as compared with mice that received no second infusion with BMC (<20% donor cells). These data indicate that skin allograft acceptance, which is a strong immunogen, is dependent on the level of hematopoietic cell chimerism in recipients.

Mixed chimeras with 20% or more donor cells in their blood accepted donor skin allografts for >270 days without any additional treatment (Table 4, group 6). Most of the mice that did not receive the second inoculum of donor BMC had less than 20% donor cells in the blood and rejected donor skin allografts (Table 4, group 5). This same group of mice nonetheless accepted other donor-derived tissues. These data demonstrate that although a relatively low number of donor cells in the blood (e.g., less than about 20%) may be sufficient for successful engraftment of marrow-derived stromal cells and heart grafts, consistent acceptance of skin allografts derived from the same donor across strong MHC barriers requires a higher level of hematopoietic cell chimerism.

### EXAMPLE 7

### Specificity of transplantation tolerance induced by the tolerogenic treatment

Tolerant BALB/c recipients with intact B6 skin allografts for 150 days rejected (11/11), within 18-20 days, a second skin allograft obtained from a third party (CBA) donor while keeping intact the original B6 skin allograft. This indicates that donor-type specific transplantation tolerance was induced and maintained in recipients capable of generating normal immune responses with full expression of alloreactivity to non-relevant transplantation antigens.

Acceptance of donor skin allografts was observed in all strain combinations investigated including, DBA→BALB/c (n=10), B6→CBA (n=3), B6→BALB/c (n=21) and BALB/c→B6 (n=9).

### EXAMPLE 8

### Application of tolerogenic treatment for induction of transplantation tolerance to skin xenografts in rat→mouse combination

Two groups of mice were administered sTLI of 6 fractions of 200 cGy/day. After sTLI conditioning, both groups were administered 30x10⁶ non-T cell depleted Lewis rat BMC intravenously. The first group was given a single dose of 200mg/kg Cy the next day. Another 3x10⁷ non T cell depleted rat BMC were administered the following day. In the second group, a dose of 60 mg/kg Cy was given daily for 3 days in contrast to the single 200 mg/kg dose given the first group. The first dose of Cy was given 10 hours after the first rat BMC inoculation, the second dose at 24 hours and the third dose at 48 hours (see below). After administration of three doses of 60 mg/kg Cy, a second inoculation of 3x10⁷ non T cell depleted rat BMC were administered.

### RESULTS

The first group of mice treated as described above (Table 5, group 1) accepted the second inoculum of 3x10⁷ non T cell depleted BMC from Lewis rats. Lethal GVHD was induced, however, in most of the recipients, suggesting fast engraftment of donor cells despite the relatively mild and non-myeloablative immunosuppressive conditioning (Table 5, group 1). Interestingly, mice that developed GVHD, indicating acceptance of donor cells, were still capable of rejecting donor-derived skin grafts prior to succumbing to the disease. These results confirm the observation that residual donor-reactive host T cells mediating host vs graft reaction may survive the immunosuppressive/tolerogenic treatment and cause rejection of highly immunogenic donor-derived skin xenografts.

**Table 5.**

| Tolerance in mice to Lewis rat bone marrow cells and skin xenografts. | | | | | | |
|---|---|---|---|---|---|---|
| Treatment of Mice After sTLI Conditioning | | | | | | |
| | Day 0 | Day 1 | Day 2 | Day 3 | Skin Graft Acceptance | Skin Xenograft Survival Time in days^{d} |
| Rat BMC^{a} | | Cy 200 mg/kg | Rat BMC^{c} | | 0/6 | <20(6) |
| Rat BMC^{a} | Cy^{b} 60 mg/kg | Cy 60 mg/kg | Cy 60 mg/kg | Rat BMC^{c} | 15/20 | >176(2), 123, >95(5) >67(3), 39(2) ,24(2)^{e} |

| | | | | | | |
|---|---|---|---|---|---|---|
| ^{a}3X10⁷ Lewis BMC intravenously. | | | | | | |
| ^{b}1st injection with Cy 10 h after the 1st rat BMC inoculation. | | | | | | |
| ^{c}3x10⁷ Lewis BMC intravenously. | | | | | | |
| ^{d}In parentheses number of mice keeping rat skin graft for indicated period. | | | | | | |
| ^{e}5/8 mice died from GVHD with rat skin xenograft accepted. Seven of 15 mice with intact skin allografts developed no acute GVHD. | | | | | | |

The results improved, in the second group, when Cy was divided into three equal doses of 60 mg/kg and injected 10 h, 24 h and 48 h after the first infusion of non-T cell depleted rat BMC. Under these conditions, 15 of 20 B6 recipients accepted full thickness Lewis rat skin xenografts (Table 2, group 2). By modifying the Cy administration protocol, host xenoreactive cells may have been more effectively controlled. Normal donor hair growth was observed in all 15 recipients, 5 of which developed lethal GVHD. The surviving mice did not develop clinical signs of GVHD although they were transplanted with non T cell depleted xenogeneic BMC suggesting that residual host-type hematopoietic cells may down-regulate donor-derived immunocompetent T cells through a veto effect.

### EXAMPLE 9

### Effect of varying doses of TLI on GVHD-free and donor-type skin allograft survival

BALB/c mice were treated with 0 to 6 doses of sTLI, with each dose being 200 cGy. After administration of sTLI, one group of mice received Cy (200mg/kg), followed, one day later, by 3 x 10⁷ or 3 x 10⁶ BMC (non-T cell-depleted) obtained from B6 donors. A second group of mice received, one day after sTLI, 3 x 10⁷ BMC (non-T cell-depleted) from B6 donors. These mice, 24 hours later, were administered Cy (200mg/kg). One day after the Cy, these mice again received 3 x 10⁷ or 3 x 10⁶ non-T cell-depleted BMC also obtained from B6 donors. A third group of mice received, after sTLI, 0.3 ml of blood from B6 donors and, 24 hours later, Cy (200mg/kg). Once again, one day after Cy, 3 x 10⁷ or 3 x 10⁶ non-T cell-depleted BMC, obtained from B6 donors, were administered to these mice. Twenty days later, donor B6 skin was grafted into surviving mice of all the groups.

Another set of experiments was conducted to correlate donor cell levels and skin allograft acceptance. The tolerogenic treatment included varying numbers of sTLI doses, followed, a day later, by 3 x 10⁷ BMC and, 24 hours later, Cy (200mg/kg). A day after administration of Cy, a second infusion of 3 x 10⁷ BMC was administered. Donor skin was grafted 20 days later. Percentages of donor blood cells in host mice were evaluated at 100-130 days after skin grafting.

In another set of experiments, sTLI-treated BALB/c mice were tolerized as indicated in Table 6 followed, 20 days later, with donor skin allografts. At either day 100 or day 120, donor cell chimerism was assayed and mixed lymphocyte reaction (MLR) tests were performed. The T cells were then enriched by lysing red blood cells with ammonium chloride, followed by passage through a nylon wool column and reconstituting in RPMI medium supplemented with 10% of AB Human serum, 0.09 mM nonessential amino acids, 1 mM sodium pyruvate, 2 mM glutamine, 100 µg/ml streptomycin and 100U/ml penicillin (Biological Industries, Beit Haemek, Israel) and 0.05 mM 3-mercaptoethanol (Sigma, USA). 10⁵ responding T cells were incubated with 10⁶ stimulating T cells (3000 cGy irradiated) in flat bottom microplates (Costar, USA) at 37°C, 5% CO₂ for 3 days. The cells were pulsed on the third day with 1 µCi [³H] thymidine and harvested on the fourth day.

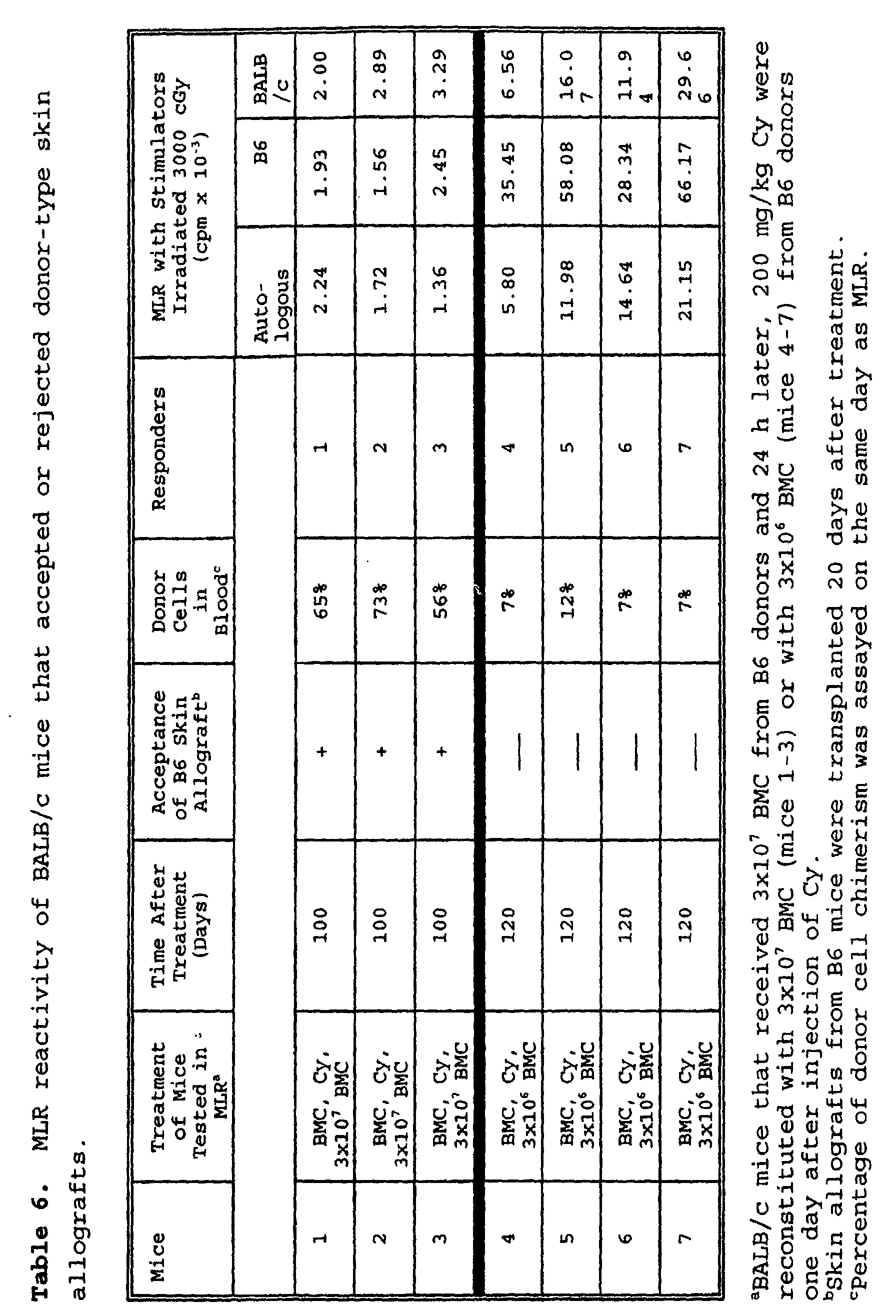

### RESULTS

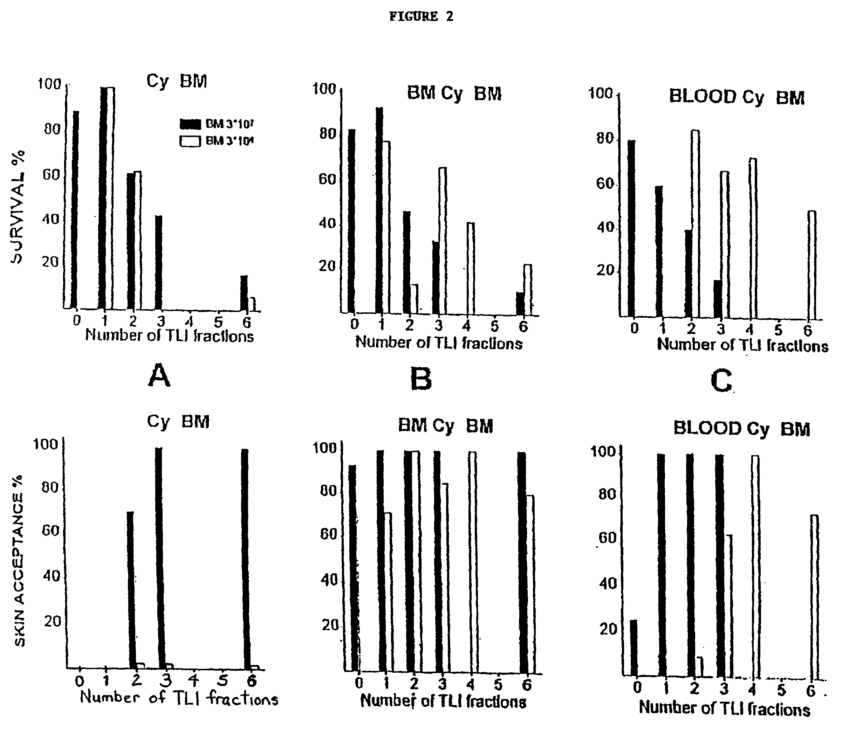

In the absence of donor-specific tolerization (Figure 2A, Group A), 0, 1, or 2 doses of sTLI appeared to be sufficient for a high probability of GVHD-free survival when 3 x 10⁷ BMC or 3 x 10⁶ BMC were administered subsequent to the Cy treatment. Administration of these low doses of sTLI to a host results in retention of relatively high numbers of host functional T cells. Consequently, the relatively high rate of GVHD-free survival at low sTLI doses may be due to a veto effect in which the levels of host- and donor-derived veto cells are in balanced equilibrium. Donor skin graft acceptance was low at 0 and 1 sTLI dose but increased at 2, 3, and 6 sTLI doses (data not available for 4 and 5 sTLI fractions). However, the GVHD-free survival rate decreased at these doses of sTLI. (Figure 2A). Thus, in the absence of donor-specific tolerization, none of the regimens led to a high percentage of GVHD-free survival and a high percentage of donor skin graft acceptance.

Donor-specific tolerization in the second group of mice (Figure 2A, Group B) resulted in high GVHD-free survival at low doses of sTLI, and even with no TLI treatment. The donor-specific tolerization also resulted in a high probability of donor skin allograft acceptance regardless of the number of sTLI doses. Without any sTLI fractions, a second dose of 3 x 10⁷ BMC appears to be necessary. The use of higher numbers of sTLI fractions resulted in higher GVHD morbidity, although skin allograft acceptance remained high. With regards to GVHD, the higher numbers of sTLI fractions seem to be more successful with a lower dose (3 x 10⁶) of BMC. This may be due to a veto effect in which lower numbers of host veto cells, due to administration of higher numbers of sTLI fractions, are in balanced equilibrium with a lower dose of BMC.

The infusion of 0.3 ml of blood, without any TLI, in the third group of mice (Figure 2C, group C) resulted in a high probability of GVHD-free survival but low donor skin allograft acceptance. This third group of mice had high GVHD-free survival when 0, 1, or 2 fractions of sTLI were administered and the mice received a large dose of (3 x 10⁷) BMC following Cy. However, higher numbers of sTLI fractions were more successful with a smaller dose of (3 x 10⁶) BMC following Cy. These results may be due to a veto effect as discussed above for the second group of mice.

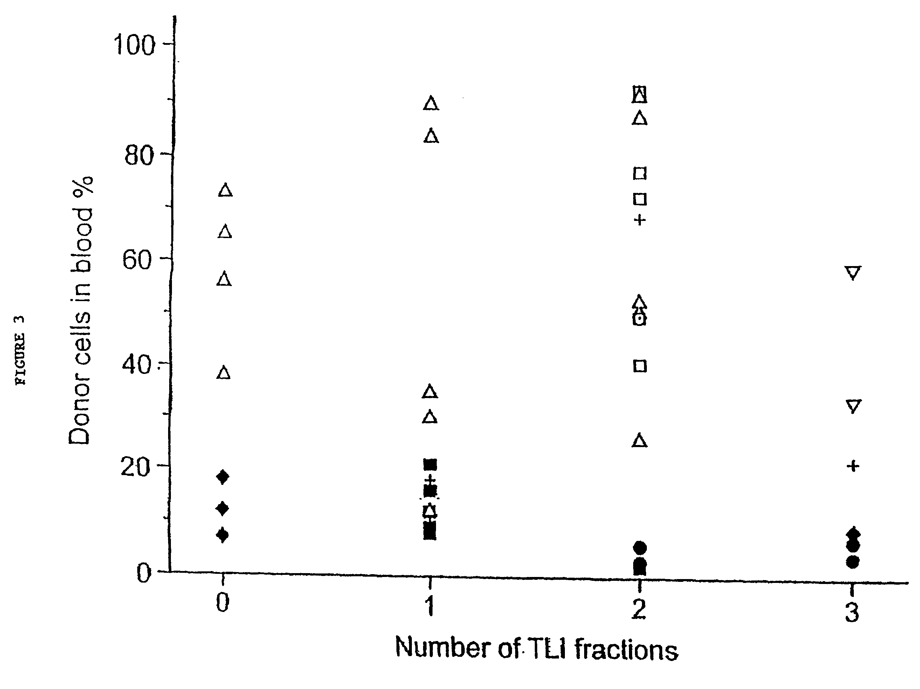

The correlation between the percentages of donor blood cells in a recipient and donor-skin allograft acceptance is illustrated in Figure 3. These experiments indicated that the percentage of donor blood cells in the recipient was critical for skin allograft acceptance. Recipients with less than 20-25% donor blood cells did not accept donor-skin allografts (solid symbols). In contrast, recipients having greater than 20-25% donor blood cells accepted donor-skin allografts (empty symbols). Furthermore, recipients were able to obtain greater that 20-25% of donor blood cells even when conditioned with 0, 1, 2, or 3 sTLI fractions in the tolerogenic treatment.

MLR reactivity data (Table 6) indicated that mice with low levels of chimerism (mice 4-7) were not completely tolerized. The responder T lymphocytes proliferated in the presence of stimulators from autologous, BALB/c and B6 sources. The response to B6 stimulators was especially high. In contrast, mice with high levels of chimerism (mice 1-3) did not respond to stimulators from any of the sources.

### EXAMPLE 10

### Non-Myeloablative, Donor-Specific Tolerogenic Treatment in a Human Patient

Patient No. 1 Prior to non-myeloablative conditioning, donor-specific tolerance induction, and allogeneic bone marrow transplantation (ABMT), this male patient underwent autologous stem cell transplantation (ASCT) almost 38 months after diagnosis of Hodgkin's Disease stage III B. The patient had failed MOPP/ABVD alternative treatment (8 cycles), radiation therapy, subsequent treatments with velban, adriamycin, bleomycin and DTIC, and repeated cycles of additional chemotherapy including, following his first overt relapse 2 years after diagnosis, MOPP (4 cycles) and VP16, cisplatin, ifosfamide, and uromitexan (5 cycles). Relapse was noted again 2 months after ASCT and the clinical picture of fever without obvious infectious etiology suggested persistence of the Hodgkin's Disease.

Allogeneic bone marrow transplantation (ABMT), following non-myeloablative conditioning and donor-specific tolerization was offered to the patient as a possible method of treatment. It was considered that this treatment could overcome long-lasting hypoplasia and could antagonize the persisting Hodgkin's Disease by inducing graft vs. Hodgkin's Disease tumor cell responses,

Tissue typing data revealed a phenotypic mismatch in HLA class I (serological testing) between the patient and the available donor, his father:

| | | | | |
|---|---|---|---|---|
| Patient | A28 | A19 | B41 | B5 |
| Donor (father) | A28 | A30 | B41 | B51 |

Typing of HLA class II revealed:

| | | | | |
|---|---|---|---|---|
| Patient | DRB1*1104 | DRB1*0404 | DQB1*0301 | DQB1*0402 |
| Donor (father) | DRB1*1101 | DRB1*0404 | DQB1*0301 | DQB1*0402 |

Starting on day 0, the patient was conditioned non-myeloablatively with Fludarabine (30mg/kg/day) for 3 consecutive days. One day later, the patient received an infusion of G-CSF mobilized peripheral blood cells ("first allograft") collected form his father (2.98 x 10⁸ nucleated cells/kg) as a source of donor-specific antigens, followed by 3 daily non-myeloablative period doses of cytoxan 60mg/kg (4,500 mg daily) to eliminate donor-specific alloreactive T cells. An infusion of unselected paternal bone marrow cells (9.6 x 10⁸ nucleated cells/kg) was carried out ("second allograft") one day after termination of the last dose of cytoxan. It was decided to use unmodified bone marrow cells with no further T cell depletion for the second allograft in order to maximize the chance of stem cell engraftment on the one hand as well as GVT effects on the other.

Fever up to almost 40°C developed in the first week after the second allograft and the patient required frequent single donor platelet infusions for prevention of bleeding. The patient also received antibiotic therapy with amikacin, tazocin, preventive therapy against fungal infection with diflucan and acyclovir therapy against cytomegalovirus infection. Since fever did not respond completely to antibiotic therapy, amphotericin B (1mg/kg) was given every other day. Fever persisted throughout hospitalization. The patient's white blood cell count (WBC) rose to 1.0 x 10⁹/L on day +14 and his absolute neutrophil count (ANC) reached ≥ 0.5 x 10⁹/L on day +14 and ≥ 1.0 x 10⁹/L on day +28. The WBC rose gradually to a maximal level of 5.1 x 10⁹/L with 75% granulocytes. However, thrombocytopenia persisted. Engraftment was confirmed by rising counts and by detection of donor DNA by the Variable Number of Tandem Repeats - Polymerase Chain Reaction (VNTR-PCR), a technique known to those in the art.

On day +10, the patient experienced a grand mal seizure which responded to valium infusion. No focal neurologic findings were found except that the Babinski's sign was positive bilaterally. Cyclosporine A was administered as a prophylactic treatment for GVHD. Overt skin rash typical of GVHD appeared on day +12. Liver manifestations developed subsequently. Despite combination therapy with solumedrol (2mg/kg) daily and cyclosporine, with continuation of the antibiotic and anti-fungal therapy, the patient's condition deteriorated gradually, with diarrhea up to 12 times a day, starting on day +16, a symptom indicative of stage IV GVHD. Despite intensive treatment of both GVHD and potential infections, spikes of fever continued with dyspnea that developed in parallel with pulmonary bleeding and bilateral interstitial infiltration in the lungs on day +28. The patient was intubated on day +29. Large volumes of secretion were aspirated through the tube. The secretions included blood but lavage did not reveal any infectious agent. Despite intensive therapy including dopamine drip and careful maintenance of pulmonary system, the blood pressure dropped gradually and the patient expired on dau +29.

In conclusion, the successful engraftment of the patient by his father's bone marrow used for the second allograft indicated that HLA mismatched stem cells can be accepted following selective depletion of host cells with the capacity to reject donor alloantigenic tissue and without myeloablative conditioning. Developments in the patient suggest that, due to pancytopenia following ASCT and the failure to establish a high level of protective mixed chimerism, he may have been more susceptible to GVHD. T cell undepleted bone marrow was used for the second allograft and this unfortunately resulted in GVHD. Nevertheless, the above-described findings demonstrated that HLA mismatched cells can be accepted and engrafted without myeloablative conditioning using the described tolerogenic protocol.

These data considered in light of murine experiments indicate that it will be possible to obtain engraftment in human patients without GVHD if the donor bone marrow used for the second is allograft is either (a) depleted of T-cells prior to infusion or (b) is used undepleted but a transient stage of mixed chimerism in the recipient is achieved. Furthermore, the combined findings of this clinical study and the murine experiments indicate that, in human patients, it will be possible to prevent rejection of allografts if the recipient is depleted of donor-specific T cells prior to the allograft.

## Claims

1. The use of donor antigens from a non-syngeneic donor in the manufacture of a medicament for inducing stable and donor-specific tolerance to non-syngeneic transplants in a host by:
(a) administering said medicament to a host mammal;
(b) administering a non-myeloablative dose of a lymphocytotoxic or tolerizing agent to said host mammal to selectively eliminate said host mammal's lymphocytes responding to said donor antigens; and
(c) administering a preparation of hematopoietic stem cells from said non-syngeneic donor to said host mammal, wherein when said hematopoietic stem cells are stem cells obtained from the liver or yolk sac of embryos, then said embryos are non-human animal embryos.

2. The use of a lymphocytotoxic or tolerizing agent in the manufacture of a medicament for inducing stable and donor-specific tolerance to non-syngeneic transplants in a host by:
(a) administering donor antigens from a non-syngeneic donor to a host mammal;
(b) administering a non-myeloablative dose of said medicament to said host mammal to selectively eliminate said host mammal's lymphocytes responding to said donor antigens; and
(c) administering a preparation of hematopoietic stem cells from said non-syngeneic donor to said host mammal, wherein when said hematopoietic stem cells are stem cells obtained from the liver or yolk sac of embryos, then said embryos are non-human animal embryos.

3. The use of hematopoietic stem cells from a non-syngeneic donor in the manufacture of a medicament for. inducing stable and donor-specific tolerance to non-syngeneic transplants in a host by:
(a) administering donor antigens from said non-syngeneic donor to said host mammal;
(b) administering a non-myeloablative dose of a lymphocytotoxic or tolerizing agent to said host mammal to selectively eliminate said host mammal's lymphocytes responding to said donor antigens; and
(c) administering said medicament to said host mammal, wherein when said heriatopoietic stem cells are stem cells obtained from the liver or yolk sac of embryos, then said embryos are non-human animal embryos.

4. The use of Claim 1, 2 or 3 further comprising prior to step (a), administering an immunosuppressive agent to said host mammal in a non-myeloablative regimen sufficient to decrease said host mammal's functional T lymphocyte population.

5. The use of Claim 1,2 or 3, wherein said donor antigens comprise one or more antigens selected from the group consisting of non-cellular antigens, cells, organs and tissues.

6. The use of Claim 1, 2 or 3, wherein said donor antigens comprise hematopoietic stem cells.

7. The use of Claim 1, 2 or 3, further comprising, following step (c), treating said host mammal with allogeneic cell therapy comprising infusing allogeneic lymphocytes from said donor into said host mammal.

8. The use of Claim 1, 2 or 3, further comprising, following step (c), transplanting cells, a tissue or an organ from said donor into said host mammal.

9. The use of Claim 1, 2 or 3, wherein said host mammal's blood possesses at least 20% donor cells resulting from performance of step (c).

10. The use of Claim 4, wherein said immunosuppresive agent is one or more agents selected from the group consisting of immunosuppresive drugs, alkylating agents, ionizing radiation and anti-leukocyte or anti-leukocyte function antibodies.

11. The use of Claim 10, wherein said immunosuppressive agent is total lymphoid irradiation (TLI), and wherein said TLI preferably comprises administration of 1-12 doses of 200 cGy.

12. The use of Claim 4, wherein said donor antigens comprise hematopoietic stem cells and said decrease of said T lymphocyte population is to a level permitting at least transient survival of said hematopoietic stem cells.

13. The use of Claim 12, wherein said decrease of said T lymphocyte population is at least about 90%, preferably at least about 95%, and more preferably at least about 99%.

14. The use of Claim 1, 2 or 3, wherein said non-syngeneic donor is an allogeneic donor.

15. The use of Claim 1, 2 or 3, wherein said non-syngeneic donor is a xenogeneic donor.

16. The use of Claim 1, 2 or 3, wherein said host mammal is a human patient, preferably a cancer patient.

17. The use of Claim 1, 2 or 3, wherein said lymphocytotoxic agent is cyclophosphamide.

18. A host-derived hematopoietic cell composition comprising host-originating and donor-originating hematopoietic cells, said composition being depleted of donor-specific, host-originating lymphocytes, and said composition being obtainable by a method comprising:
(a) administering donor antigens from a non-syngeneic donor to a host mammal;
(b) administering a non-myeloablative dose of lymphocytotoxic or tolerizing agent to said host mammal to selectively eliminate said host mammal's lymphocytes responding to said donor antigens;
(c) administering a preparation of hematopoietic stem cells from said non-syngeneic donor to said host mammal; and
(d) isolating said hematopoietic cell composition from said host mammal, wherein when said hematopoietic stem cells are stem cells obtained from the liver of yolk sac of embryos, then said embryos are non-human animal embryos.

19. A non-human mammal/human chimera obtainable by a method comprising:
(a) administering antigens from a human donor to a host non-human mammal;
(b) administering a non-myeloablative dose of lymphocytotoxic or tolerizing agent to said host mammal to selectively eliminate said host mammal's lymphocytes responding to said antigens; and
(c) administering a preparation of hematopoietic stem cells from said human donor to said host mammal, wherein said hematopoietic stem cells from said human donor are not stem cells obtained from the liver or yolk sac of embryos.

20. A host derived hematopoietic cell composition according to Claim 18 or a chimera according to Claim 19, wherein the method further comprises, prior to step (a), administering an immunosuppressive agent to said host mammal in a non-myeloablative regimen sufficient to decrease said host mammal's functional T lymphocyte population.

21. A non-human mammal/human chimera according to Claim 19, wherein said host mammal is a rodent or a pig.

22. A rodent stably engrafted with human hematopoietic stem cells, said rodent constituting a hematopoietic mixed chimera, wherein said hematopoietic mixed chimera is obtainable by a method comprising:
(a) administering antigens from a human donor to an individual rodent;
(b) administering a non-myeloablative dose of a lymphocytotoxic or tolerizing agent to said individual rodent to selectively eliminate said individual rodent's lymphocytes responding to said antigens; and
(c) administering a preparation of hematopoietic stem cells from said human donor to said individual rodent to create said mixed chimera,
wherein said mixed chimera comprises hematopoietic cells originating from said individual rodent and from said human donor and said hematopoietic cells originating from said individual rodent are depleted of lymphocytes specific for antigens of said human donor, and wherein said hematopoietic stem cells from said human donor are not stem cells obtained from the liver or yolk sac of embryos.

23. A pig stably engrafted with human hematopoietic stem cells, said pig constituting a hematopoietic mixed chimera, wherein said hematopoietic mixed chimera is obtainable by a method comprising:
(a) administering antigens from a human donor to an individual pig;
(b) administering a non-myeloablative dose of a lymphocytotoxic or tolerizing agent to said individual pig to selectively eliminate said individual pig's lymphocytes responding to said antigens; and
(c) administering a preparation of hematopoietic stem cells from said human donor to said individual pig to create said mixed chimera,
wherein said mixed chimera comprises hematopoietic cells originating from said individual pig and from said human donor and said hematopoietic cells originating from said individual pig are depleted of lymphocytes specific for antigens of said human donor, and wherein said hematopoietic stem cells from said human donor are not stem cells obtained from the liver or yolk sac of embryos.

24. The use of an immunosuppresive agent in the preparation of a medicament, for inducing stable and donor-specific tolerance to non-syngeneic transplants in a host by:
(a) administering said medicament to said host mammal in a non-myeloablative regimen sufficient to decrease said host mammal's functional T lymphocyte population;
(b) administering donor antigens from a non-syngeneic donor to said host mammal;
(c) administering a non-myeloablative dose of a lymphocytotoxic or tolerizing agent to said host mammal to selectively eliminate said host mammal's lymphocytes responding to said donor antigens; and
(d) administering a preparation of hematopoietic stem cells from said non-syngeneic donor to said host mammal, wherein when said hematopoietic stem cells are stem cells obtained from the liver or yolk sac of embryos, then said embryos are non-human animal embryos.

## Patentansprüche

1. Verwendung von Spender-Antigenen von einem nicht-syngenen Spender bei der Herstellung eines Medikaments, um in einem Wirt eine stabile und spenderspezifische Toleranz für nicht-syngene Transplantate zu induzieren, durch:
(a) Verabreichen des Medikaments an einen Wirtssäuger;
(b) Verabreichen einer nicht-myeloablativen Dosis eines lymphozytotoxischen oder tolerisierenden Mittels an den Wirtssäuger, um die auf die Spender-Antigene ansprechenden Lymphozyten des Wirtssäugers selektiv zu eliminieren; und
(c) Verabreichen einer Zubereitung von hämopoetischen Stammzellen von dem nicht-syngenen Spender an den Wirtssäuger, wobei dann, wenn die hämopoetischen Stammzellen Stammzellen sind, die aus der Leber oder dem Dottersack von Embryos gewonnen sind, die besagten Embryos nicht-humane tierische Embryos sind.

2. Verwendung eines lymphozytotoxischen oder tolerisierenden Mittels bei der Herstellung eines Medikaments, um in einem Wirt eine stabile und spenderspezifische Toleranz für nicht-syngene Transplantate zu induzieren, durch:
(a) Verabreichen von Spender-Antigenen von einem nicht-syngenen Spender an einen Wirtssäuger;
(b) Verabreichen einer nicht-myeloablativen Dosis des Medikaments an den Wirtssäuger, um die auf die Spender-Antigene ansprechenden Lymphozyten des Wirtssäugers selektiv zu eliminieren; und
(c) Verabreichen eines Präparats von hämopoetischen Stammzellen von dem nicht-syngenen Spender an den Wirtssäuger, wobei dann, wenn die hämopoetischen Stammzellen Stammzellen sind, die aus der Leber oder dem Dottersack von Embryos gewonnen sind, die besagten Embryos nicht-humane tierische Embryos sind.

3. Verwendung von hämopoetischen Stammzellen von einem nicht-syngenen Spender bei der Herstellung eines Medikaments, um in einem Wirt eine stabile und spenderspezifische Toleranz für nicht-syngene Transplantate zu induzieren, durch:
(a) Verabreichen von Spender-Antigenen von dem nicht-syngenen Spender an den Wirtssäuger;
(b) Verabreichen einer nicht-myeloablativen Dosis eines lymphozytotoxischen oder tolerisierenden Mittels an den Wirtssäuger, um die auf die Spender-Antigene ansprechenden Lymphozyten des Wirtssäugers selektiv zu eliminieren; und
(c) Verabreichen des Medikaments an den Wirtssäuger, wobei dann, wenn die hämopoetischen Stammzellen Stammzellen sind, die aus der Leber oder dem Dottersack von Embryos gewonnen sind, die besagten Embryos nicht-humane tierische Embryos sind.

4. Verwendung nach Anspruch 1, 2 oder 3, die ferner vor Schritt (a) aufweist: Verabreichen eines Immunsuppressivums an den Wirtssäuger in einer nicht-myeloablativen Verordnung, die ausreichend ist, um die funktionelle T-Lymphozytenpopulation des Wirtssäugers zu verringern.

5. Verwendung nach Anspruch 1, 2 oder 3, wobei die Spender-Antigene eines oder mehrere Antigene aufweisen, die aus der Gruppe ausgewählt sind, die aus nicht-zellulären Antigenen, Zellen, Organen und Geweben besteht.

6. Verwendung nach Anspruch 1, 2 oder 3, wobei die Spender-Antigene hämopoetische Stammzellen aufweisen.

7. Verwendung nach Anspruch 1, 2 oder 3, die ferner im Anschluß an Schritt (c) aufweist: Behandeln des Wirtssäugers mit einer allogenen Zelltherapie, die das Infundieren von allogenen Lymphozyten von dem Spender in den Wirtssäuger aufweist.

8. Verwendung nach Anspruch 1, 2 oder 3, die ferner im Anschluß an Schritt (c) aufweist: Transplantieren von Zellen, eines Gewebes oder eines Organs von dem Spender in den Wirtssäuger.

9. Verwendung nach Anspruch 1, 2 oder 3, wobei das Blut des Wirtssäugers mindestens 20 % Spenderzellen aufweist, die aus der Durchführung von Schritt (c) resultieren.

10. Verwendung nach Anspruch 4, wobei das Immunsuppressivum eines oder mehrere Mittel ist, die aus der Gruppe ausgewählt sind, die aus immunsuppressiven Substanzen, Alkylanzien, ionisierender Strahlung und Anti-Leukozyt- oder Anti-Leukozytfunktions-Antikörpern besteht.

11. Verwendung nach Anspruch 10, wobei das Immunsuppressivum totale Lymphoid-Bestrahlung (TLI) ist und wobei die TLI bevorzugt die Verabreichung von 1 bis 12 Dosen von 200 cGy aufweist.

12. Verwendung nach Anspruch 4, wobei die Spender-Antigene hämopoetische Stammzellen aufweisen und die genannte Verringerung der T-Lymphozytenpopulation auf einen Wert erfolgt, der zumindest das übergangsweise Überleben der hämopoetischen Stammzellen zuläßt.

13. Verwendung nach Anspruch 12, wobei die Verringerung der T-Lymphozytenpopulation mindestens ungefähr 90 %, bevorzugt mindestens ungefähr 95 % und noch stärker bevorzugt mindestens ungefähr 99 % beträgt.

14. Verwendung nach Anspruch 1, 2 oder 3, wobei der nicht-syngene Spender ein allogener Spender ist.

15. Verwendung nach Anspruch 1, 2 oder 3, wobei der nicht-syngene Spender ein xenogener Spender ist.

16. Verwendung nach Anspruch 1, 2 oder 3, wobei der Wirtssäuger ein Humanpatient, bevorzugt ein Krebspatient ist.

17. Verwendung nach Anspruch 1, 2 oder 3, wobei das lymphozytotoxische Mittel Cyclophosphamid ist.

18. Von einem Wirt abgeleitete hämopoetische Zellzusammensetzung, die von dem Wirt stammende und von einem Spender stammende hämopoetische Zellen aufweist, wobei die Zusammensetzung in bezug auf spenderspezifische, vom Wirt stammende Lymphozyten verarmt ist und die Zusammensetzung durch ein Verfahren zu erhalten ist, das folgendes aufweist:
(a) Verabreichen von Spender-Antigenen von einem nicht-syngenen Spender an einen Wirtssäuger;
(b) Verabreichen einer nicht-myeloablativen Dosis von lymphozytotoxischem oder tolerisierendem Mittel an den Wirtssäuger, um die auf die Spender-Antigene ansprechenden Lymphozyten des Wirtssäugers selektiv zu eliminieren;
(c) Verabreichen einer Zubereitung von hämopoetischen Stammzellen von dem nicht-syngenen Spender an den Wirtssäuger; und
(d) Isolieren der hämopoetischen Zellzusammensetzung von dem Wirtssäuger,
wobei dann, wenn die hämopoetischen Stammzellen Stammzellen sind, die aus der Leber oder dem Dottersack von Embryos gewonnen sind, die besagten Embryos nicht-humane tierische Embryos sind.

19. Nicht-humane Säuger-/Human-Chimäre, erhältlich durch ein Verfahren, das folgendes aufweist:
(a) Verabreichen von Antigenen von einem Humanspender an einen nichthumanen Wirtssäuger;
(b) Verabreichen einer nicht-myeloablativen Dosis von lymphozytotoxischem oder tolerisierendem Mittel an den Wirtssäuger, um die auf die Antigene ansprechenden Lymphozyten des Wirtssäugers selektiv zu eliminieren; und
(c) Verabreichen einer Zubereitung von hämopoetischen Stammzellen von dem Humanspender an den Wirtssäuger, wobei die hämopoetischen Stammzellen von dem Humanspender nicht Stammzellen sind, die aus der Leber oder dem Dottersack von Embryos erhalten sind.

20. Von einem Wirt abgeleitete hämopoetische Zellzusammensetzung nach Anspruch 18 oder Chimäre nach Anspruch 19, wobei das Verfahren ferner vor Schritt (a) aufweist: Verabreichen eines Immunsuppressivums an den Wirtssäuger in einer nicht-myeloablativen Verordnung, die ausreichend ist, um die funktionale T-Lymphozytenpopuiation des Wirtssäugers zu verringern.

21. Nicht-humane Säuger-/Human-Chimäre nach Anspruch 19, wobei der Wirtssäuger ein Nager oder ein Schwein ist.

22. Nager, in den hämopoetische Humanstammzellen stabil transplantiert sind, wobei der Nager eine hämopoetische Misch-Chimäre bildet und wobei die hämopoetische Misch-Chimäre durch ein Verfahren zu erhalten ist, das folgendes aufweist:
(a) Verabreichen von Antigenen von einem Human-Spender an einen individuellen Nager;
(b) Verabreichen einer nicht-myeloablativen Dosis eines lymphozytotoxischen oder tolerisierenden Mittels an den individuellen Nager, um auf die Antigene ansprechende Lymphozyten des individuellen Nagers selektiv zu eliminieren; und
(c) Verabreichen einer Zubereitung von hämopoetischen Stammzellen von dem Human-Spender an den individuellen Nager, um die Misch-Chimäre zu erschaffen,
wobei die Misch-Chimäre hämopoetische Zellen aufweist, die von dem individuellen Nager und von dem Human-Spender stammen, und die von dem individuellen Nager stammenden hämopoetischen Zellen hinsichtlich Lymphozyten, die für Antigene des Human-Spenders spezifisch sind, verarmt sind, und wobei die hämopoetischen Zellen von dem Human-Spender nicht Stammzellen sind, die aus der Leber oder dem Dottersack von Embryos erhalten sind.

23. Schwein, in das hämopoetische Humanstammzellen stabil transplantiert sind, wobei das Schwein eine hämopoetische Misch-Chimäre bildet und wobei die hämopoetische Misch-Chimäre durch ein Verfahren zu erhalten ist, das folgendes aufweist:
(a) Verabreichen von Antigenen von einem Human-Spender an ein individuelles Schwein;
(b) Verabreichen einer nicht-myeloablativen Dosis eines lymphozytotoxischen oder tolerisierenden Mittels an das individuelle Schwein, um auf die Antigene ansprechende Lymphozyten des individuellen Schweins selektiv zu eliminieren; und
(c) Verabreichen einer Zubereitung von hämopoetischen Stammzellen von dem Human-Spender an das individuelle Schwein, um die Misch-Chimäre zu erschaffen,
wobei die Misch-Chimäre hämopoetische Zellen aufweist, die von dem individuellen Schwein und von dem Human-Spender stammen, und die von dem individuellen Schwein stammenden hämopoetischen Zellen hinsichtlich Lymphozyten, die für Antigene des Human-Spenders spezifisch sind, verarmt sind, und wobei die hämopoetischen Zellen von dem Human-Spender nicht Stammzellen sind, die aus der Leber oder dem Dottersack von Embryos erhalten sind.

24. Verwendung eines Immunsuppressivums bei der Zubereitung eines Medikaments, um in einem Wirt eine stabile und spenderspezifische Toleranz für nicht-syngene Transplantate zu induzieren, durch:
(a) Verabreichen des Medikaments an den Wirtssäuger in einer nicht-myeloablativen Verordnung, die ausreichend ist, um die funktionelle T-Lymphozytenpopulation des Wirtssäugers zu verringern;
(b) Verabreichen von Spender-Antigenen von einem nicht-syngenen Spender an den Wirtssäuger;
(c) Verabreichen einer nicht-myeloablativen Dosis eines lymphozytotoxischen oder tolerisierenden Mittels an den Wirtssäuger, um auf die Spender-Antigene ansprechende Lymphozyten des Wirtssäugers selektiv zu eliminieren; und
(d) Verabreichen einer Zubereitung von hämopoetischen Stammzellen von dem nicht-syngenen Spender an den Wirtssäuger, wobei dann, wenn die hämopoetischen Stammzellen Stammzellen sind, die aus der Leber oder dem Dottersack von Embryos erhalten sind, diese Embryos nicht-humane tierische Embryos sind.

## Revendications

1. Utilisation d'antigènes de donneur provenant d'un donneur non-syngénique dans la préparation d'un médicament pour induire une tolérance stable et spécifique vis-à-vis du donneur à des transplants non-syngéniques chez un hôte par :
(a) l'administration à un mammifère hôte dudit médicament ;
(b) l'administration audit mammifère hôte d'une dose non-myéloablative d'un agent lymphocytotoxique ou promoteur de tolérance (en anglais : "tolerizing") pour éliminer sélectivement les lymphocytes dudit mammifère hôte sensibles auxdits antigènes de donneur ; et,
(c) l'administration audit mammifère hôte d'une préparation de cellules souches hématopoïétiques provenant dudit donneur non-syngénique, dans laquelle, quand lesdites cellules souches hématopoïétiques sont des cellules souches obtenues à partir de foie ou de sac vitellin d'embryons, alors lesdits embryons sont des embryons d'animaux non-humains.

2. Utilisation d'un agent lymphocytotoxique ou promoteur de tolérance dans la préparation d'un médicament pour induire une tolérance stable et spécifique vis-à-vis du donneur à des transplants non-syngéniques chez un hôte par :
(a) l'administration à un mammifère hôte d'antigènes de donneur provenant d'un donneur non-syngénique ;
(b) l'administration audit mammifère hôte d'une dose non-myéloablative dudit médicament pour éliminer sélectivement les lymphocytes dudit mammifère hôte sensibles auxdits antigènes de donneur ; et,
(c) l'administration audit mammifère hôte d'une préparation de cellules souches hématopoïétiques provenant dudit donneur non-syngénique, dans laquelle, quand lesdites cellules souches hématopoïétiques sont des cellules souches obtenues à partir de foie ou de sac vitellin d'embryons, alors lesdits embryons sont des embryons d'animaux non-humains.

3. Utilisation de cellules souches hématopoïétiques provenant d'un donneur non-syngénique dans la préparation d'un médicament pour induire une tolérance stable et spécifique vis-à-vis du donneur à des transplants non-syngéniques chez un hôte par :
(a) l'administration à un mammifère hôte d'antigènes de donneur provenant dudit donneur non-syngénique;
(b) l'administration audit mammifère hôte d'une dose non-myéloablative d'un agent lymphocytotoxique ou promoteur de tolérance pour éliminer sélectivement les lymphocytes dudit mammifère hôte sensibles auxdits antigènes de donneur ; et,
(c) l'administration audit mammifère hôte dudit médicament, dans laquelle, quand lesdites cellules souches hématopoïétiques sont des cellules souches obtenues à partir de foie ou de sac vitellin d'embryons, alors lesdits embryons sont des embryons d'animaux non-humains.

4. Utilisation suivant la revendication 1, 2 ou 3, qui comprend en outre, avant le stade (a), l'administration audit mammifère hôte d'un agent immunosupresseur dans un régime non-myéloablatif suffisant pour diminuer la population fonctionnelle de lymphocytes T.

5. Utilisation suivant la revendication 1, 2 ou 3, dans laquelle lesdits antigènes de donneur comprennent un ou plusieurs antigènes choisis parmi l'ensemble constitué par les antigènes non-cellulaires, les cellules, les organes et les tissus.

6. Utilisation suivant la revendication 1, 2 ou 3, dans laquelle lesdits antigènes de donneur comprennent des cellules souches hématopoïétiques.

7. Utilisation suivant la revendication 1, 2 ou 3, qui comprend en outre, après le stade (c), le traitement dudit mammifère hôte par une thérapie cellulaire allogénique comprenant l'apport (en anglais : "infusion") de lymphocytes allogéniques dudit donneur chez ledit mammifère hôte.

8. Utilisation suivant la revendication 1, 2 ou 3, qui comprend en outre, après le stade (c), la transplantation de cellules d'un tissu ou d'un organe provenant dudit donneur chez ledit mammifère hôte.

9. Utilisation suivant la revendication 1, 2 ou 3, dans laquelle le sang dudit mammifère hôte possède au moins 20 % de cellules du donneur résultant de la mise en oeuvre du stade (c).

10. Utilisation suivant la revendication 4, dans laquelle ledit agent immunosuppresseur est l'un ou plusieurs des agents choisis parmi l'ensemble constitué par les substances immunosuppressives, les agents d'alkylation, une radiation ionisante et des anticorps anti-leucocyte ou à fonction anti-leucocyte.

11. Utilisation suivant la revendication 10, dans laquelle ledit agent immunosuppresseur est une irradiation lymphoïde totale (TLI), et dans laquelle ladite LTI comprend l'administration de 1-12 doses de 200 cGy.

12. Utilisation suivant la revendication 4, dans laquelle lesdits antigènes de donneur comprennent des cellules souches hématopoïétiques et la diminution de ladite la population de lymphocytes T est à un niveau permettant au moins la survie temporaire desdites cellules souches hématopoïétiques.

13. Utilisation suivant la revendication 12, dans laquelle ladite diminution de ladite la population de lymphocytes T est d'au moins 90 % environ, de préférence d'au moins 95 % environ, et mieux d'au moins 99 % environ.

14. Utilisation suivant la revendication 1, 2 ou 3, dans laquelle ledit donneur non-syngénique est un donneur allogénique.

15. Utilisation suivant la revendication 1, 2 ou 3, dans laquelle ledit donneur non-syngénique est un donneur xénogénique.

16. Utilisation suivant la revendication 1, 2 ou 3, dans laquelle ledit mammifère hôte est un patient humain, de préférence un patient cancéreux.

17. Utilisation suivant la revendication 1, 2 ou 3, dans laquelle ledit agent lymphocytotoxique est un cyclophosphamide.

18. Composition cellulaire hématopoïétique dérivant d'un hôte, comprenant des cellules hématopoïétiques originaires de l'hôte et originaires du donneur, ladite composition étant appauvrie en lymphocytes spécifiques vis-à-vis du donneur, originaires de l'hôte, et ladite composition pouvant être obtenue par un procédé comprenant:
(a) l'administration à un mammifère hôte d'antigènes de donneur provenant d'un donneur non-syngénique ;
(b) l'administration audit mammifère hôte d'une dose non-myéloablative d'un agent lymphocytotoxique ou promoteur de tolérance pour éliminer sélectivement les lymphocytes dudit mammifère hôte sensibles auxdits antigènes de donneur ;
(c) l'administration audit mammifère hôte d'une préparation de cellules souches hématopoïétiques provenant dudit donneur non-syngénique ; et,
(d) l'isolation de ladite composition cellulaire hématopoïétique à partir dudit mammifère hôte , dans laquelle, quand lesdites cellules souches hématopoïétiques sont des cellules souches obtenues à partir de foie ou de sac vitellin d'embryons, alors lesdits embryons sont des embryons d'animaux non-humains.

19. Chimère mammifère/homme non-humaine susceptible d'être obtenue par un procédé comprenant :
(a) l'administration à un mammifère hôte non-humain d'antigènes provenant d'un donneur humain ;
(b) l'administration audit mammifère hôte d'une dose non-myéloablative d'agent lymphocytotoxique ou promoteur de tolérance pour éliminer sélectivement les lymphocytes dudit mammifère hôte sensibles auxdits antigènes ; et,
(c) l'administration audit mammifère hôte d'une préparation de cellules souches hématopoïétiques provenant dudit donneur humain, dans laquelle lesdites cellules souches hématopoïétiques dudit donneur humain ne sont pas des cellules souches obtenues à partir de foie ou de sac vitellin d'embryons.

20. Composition cellulaire hématopoïétique dérivant d'un hôte selon la revendication 18 ou chimère selon la revendication 19, dans laquelle le procédé comprend en outre, avant le stade (a), l'administration audit mammifère hôte d'un agent immunosupresseur dans un régime non-myéloablatif suffisant pour diminuer la population fonctionnelle de lymphocytes T.

21. Chimère mammifère/homme non-humaine suivant la revendication 19, dans laquelle ledit mammifère hôte est un rongeur ou un porc.

22. Rongeur stablement greffé avec des cellules souches hématopoïétiques humaines, ledit rongeur constituant une chimère hématopoïétique mixte, ladite chimère hématopoïétique mixte pouvant être obtenue selon un procédé comprenant :
(a) l'administration à un rongeur individuel (en anglais : "an individual rodent") d'antigènes provenant d'un donneur humain ;
(b) l'administration audit rongeur individuel d'une dose non-myéloablative d'un agent lymphocytotoxique ou promoteur de tolérance pour éliminer sélectivement les lymphocytes dudit rongeur individuel sensibles auxdits antigènes ; et,
(c) l'administration audit rongeur individuel d'une préparation de cellules souches hématopoïétiques provenant dudit donneur humain pour créer ladite chimère mixte,
ladite chimère mixte comprenant des cellules hématopoïétiques originaires dudit rongeur individuel et dudit donneur humain, lesdites cellules hématopoïétiques originaires dudit rongeur individuel étant appauvries en lymphocytes spécifiques pour les antigènes dudit donneur humain, et lesdites cellules souches hématopoïétiques dudit donneur humain n'étant pas des cellules souches obtenues à partir de foie ou de sac vitellin d'embryons.

23. Porc stablement greffé avec des cellules souches hématopoïétiques humaines, ledit porc constituant une chimère hématopoïétique mixte, ladite chimère hématopoïétique mixte pouvant être obtenue selon un procédé comprenant :
(a) l'administration à un porc individuel (en anglais : "an individual pig") d'antigènes provenant d'un donneur humain ;
(b) l'administration audit porc individuel d'une dose non-myéloablative d'un agent lymphocytotoxique ou promoteur de tolérance pour éliminer sélectivement les lymphocytes dudit porc individuel sensibles auxdits antigènes ; et,
(c) l'administration audit porc individuel d'une préparation de cellules souches hématopoïétiques provenant dudit donneur humain pour créer ladite chimère mixte,
ladite chimère mixte comprenant des cellules hématopoïétiques originaires dudit porc individuel et dudit donneur humain, lesdites cellules hématopoïétiques originaires dudit porc individuel étant appauvries en lymphocytes spécifiques pour les antigènes dudit donneur humain, et lesdites cellules souches hématopoïétiques dudit donneur humain n'étant pas des cellules souches obtenues à partir de foie ou de sac vitellin d'embryons.

24. Utilisation d'un agent immunosuppresseur dans la préparation d'un médicament pour induire une tolérance stable et spécifique vis-à-vis du donneur à des transplants non-syngéniques chez un hôte par :
(a) l'administration à un mammifère hôte dudit médicament dans un régime non-myéloablatif suffisant pour diminuer la population fonctionnelle de lymphocytes T dudit mammifère hôte ;
(b) l'administration audit mammifère hôte d'antigènes de donneur provenant d'un donneur non-syngénique ;
(c) l'administration audit mammifère hôte d'une dose non-myéloablative d'un agent lymphocytotoxique ou promoteur de tolérance pour éliminer sélectivement les lymphocytes dudit mammifère hôte sensibles auxdits antigènes du donneur ; et,
(d) l'administration audit mammifère hôte d'une préparation de cellules souches hématopoïétiques provenant dudit donneur non-syngénique, dans laquelle, quand lesdites cellules souches hématopoïétiques sont des cellules souches obtenues à partir de foie ou de sac vitellin d'embryons, alors lesdits embryons sont des embryons d'animaux non-humains.
